(19) Europäisches Patentamt
European Patent Office
Office européen des brevets

(11) **EP 4 144 753 A1**

(12) **EUROPEAN PATENT APPLICATION**
published in accordance with Art. 153(4) EPC

(43) Date of publication:
**08.03.2023 Bulletin 2023/10**

(21) Application number: **21796985.6**

(22) Date of filing: **07.04.2021**

(51) International Patent Classification (IPC):
**C07K 14/005** $^{(2006.01)}$ **C12M 1/00** $^{(2006.01)}$
**C12N 7/00** $^{(2006.01)}$

(52) Cooperative Patent Classification (CPC):
**C07K 14/005; C12M 1/00; C12N 7/00**

(86) International application number:
**PCT/KR2021/004356**

(87) International publication number:
**WO 2021/221338 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.04.2020 KR 20200052700**
**29.04.2020 KR 20200052708**

(71) Applicant: **SK Bioscience Co., Ltd.**
**Bundang-gu, Seongnam-si,**
**Gyeonggi-do, 13494 (KR)**

(72) Inventors:
• **JUNG, Hwan-ui**
**Seongnam-si, Gyeonggi-do 13494 (KR)**
• **KIM, Yun-hee**
**Seongnam-si, Gyeonggi-do 13494 (KR)**
• **KIM, Hun**
**Seongnam-si, Gyeonggi-do 13494 (KR)**
• **PARK, Yong Wook**
**Seongnam-si, Gyeonggi-do 13494 (KR)**

(74) Representative: **Vossius & Partner**
**Patentanwälte Rechtsanwälte mbB**
**Siebertstraße 3**
**81675 München (DE)**

(54) **INFLUENZA VIRUS PRODUCTION METHOD USING DISPOSABLE CULTURE PROCESS SYSTEM, AND TEST FOR QUICKLY CHECKING CONDITIONS FOR INFLUENZA VIRUS ANTIGEN PURIFICATION**

(57) The present invention relates to an influenza virus production method using a disposable culture process system, and a test for quickly checking conditions for influenza virus antigen purification. According to the present invention, conditions for influenza surface antigen obtainment (purification) may be quickly and reliably checked according to the unique method of the present invention, even without using the single radial immunodiffusion technique which is conventionally used as a standard test method when producing influenza vaccines, and thus the production time for an influenza surface antigen subunit vaccine is notably reduced, thereby enabling quick response as a result of rapid vaccine development/manufacturing, even in a rapid novel influenza pandemic situation. In addition, according to the influenza virus production method of the present invention, culture media exchange may be carried out in an airtight system by using a continuous low-speed centrifuge using a disposable bag, and thus the possibility of contamination occurring during the virus production process may be greatly reduced.

[FIG. 1]

EP 4 144 753 A1

**Description**

**[Technical Field]**

This application claims the benefit of priority of Korean Patent Application No. 10-2020-0052700 filed on April 29, 2020 and Korean Patent Application No. 10-2020-0052708 filed on April 29, 2020, the contents of which are incorporated herein by reference in their entirety.

The present invention relates to an influenza virus production method using a single-use culture process system and a rapid confirmation test of influenza virus antigen purification conditions.

**[Background Art]**

For influenza-related diseases, treatment is important, but prevention through vaccination is the most realistic and effective approach. When vaccine development is made more rapidly, it is expected that the damage can be reduced to such an extent during new influenza pandemics.

Since the recommended strain of influenza virus for vaccine production changes every year, and because of the nature of new influenza outbreaks, where the number of infections worldwide increases at an unspecified time, the key to developing an influenza virus vaccine is to establish the conditions for mass production in a short time and to produce the vaccine with high yield and high purity.

The main antigen of influenza vaccines is hemagglutinin (HA) in the influenza virus envelope, and the potency of vaccines is also based on the concentration of hemagglutinin. Currently, in order to measure the hemagglutinin concentration in influenza vaccines, the single radial immunodiffusion technique (SRID) recommended by the European Pharmacopoeia and the World Health Organization (WHO) is commonly used at home and abroad (Non-Patent Document 1). When the hemagglutinin content is measured by the single radial immunodiffusion technique, standard antigens and standard antibodies provided from the National Institute for Biological Standards and Control (NIBSC) are used. The concentration of hemagglutinin in the influenza vaccine is measured by comparing and converting the size of the ring in which the influenza vaccine antigen and the standard antibody are aggregated with the size of the ring in which the standard antigen and the standard antibody are aggregated. However, this single radial immunodiffusion technique requires more than 24 hours to process a sample, and is greatly labor intensive compared to the throughput since most processes are manual.

A pandemic is a situation in which an infectious disease spreads around the world. In a pandemic situation, vaccines must be rapidly developed, the content of hemagglutinin must also be measured rapidly when a vaccine is developed to cope with the influenza pandemic situation. In Korea, during the unexpected H1N1 pandemic in 2009, vaccine manufacture and follow-up clinical trials were generally delayed due to the delay in the supply of standard antigens and standard antibodies from the World Health Organization (WHO). When rapid development of a vaccine is required, such as in a pandemic situation, a rapid method for measuring the hemagglutinin content is required, but it takes 2 to 3 months to develop the standard antigen and standard antibody used in the single radial immunodiffusion technique, and this has become an obstacle to the rapid development of vaccines.

Recently, studies on new physicochemical test methods have begun. In order for the newly attempted physicochemical test methods to replace the single radial immunodiffusion test, it is necessary to establish and certify data that show any correlation between the results of alternative test methods and the results of the single radial immunodiffusion technique, and test methods.

Currently, reverse-phase-high-performance liquid chromatography (RP-HPLC), isotope-dilution liquid chromatography-mass spectrometry (ID-LC/MS), deglycosylation antigen SDS-PAGE analysis, and the like are known as alternative test methods, but it is known that some methods still require standard antigens, require expensive equipment and professional manpower, or have disadvantages such as low analytical sensitivity (Non-Patent Document 2).

There is still a need in the art for methods that can easily replace the SRID technique or manufacture a vaccine is required to be manufactured without the SRID technique.

Meanwhile, influenza vaccines have been manufactured using fertilized eggs for the past 50 years. However, this manufacturing method requires about one fertilized egg to manufacture a vaccine for a single dose, so it takes a long time to manufacture the vaccine, and the manufacturing process is complicated. In addition, for vaccine manufacture, quality-controlled SPF (specific pathogen free) fertilized eggs are to be used, but the supply thereof is limited, and if it is not planned in advance, it is difficult to increase the supply of fertilized eggs when there is a sudden demand. Moreover, vaccines produced using fertilized eggs contain trace amounts of egg-derived protein, which increases the potential for allergic reactions in those susceptible to this protein. Influenza viruses sometimes undergo antigenic mutation when cultivated in fertilized eggs, and some viruses are rather incapable of being cultivated in fertilized eggs or are lethal to chickens in some cases. These disadvantages may be a major problem in supplying sufficient vaccines in the event of a global pandemic of influenza virus. In order to overcome these disadvantages, a technology for cultivating an influenza virus using an animal cell culture began about 15 years ago.

Meanwhile, cell culture for industrial-scale virus production is generally conducted using a stainless steel fermenter facility, but when such a facility is used, a process of washing and sterilizing the fermenter before use is required, so the process preparation time is long, and there is a high likelihood of contamination with external substances. In addition, cross-contamination between lots or virus strains may occur during the washing process after use. In particular, the influenza vaccine is a trivalent or tetravalent vaccine, and thus three or four different viruses must be cultivated, and there is a high probability of cross-contamination between strains, so there is a need for a new virus production process in which these problems are resolved.

Korean Patent No. 10-1464783 discloses a method for culturing MDCK cells in a single-use bioreactor, but in the method provided by the document, cell culture is performed in the presence of microcarriers, and a process of removing microcarriers is added, and thus the procedure is cumbersome, and there is a high likelihood of contamination.

[Disclosure]

[Technical Problem]

Therefore, the present inventors have studied to set the process conditions during vaccine production without using the single radial immunodiffusion technique, and confirmed that it is possible to rapidly and reliably confirm the conditions for obtaining the influenza surface antigen according to the method unique to the present invention, and in turn, the production period of a vaccine containing an influenza antigen is significantly shortened, thereby completing the present invention.

As a result of repeated intensive studies to develop a cell culture system that shortens the process preparation time and has remarkably low concerns about contamination in cell culture for influenza virus propagation, it has been found out that the object is achieved when cells are cultured in a single-use cell incubator using a single-use bag and then medium exchange before virus infection is performed using a low-speed continuous centrifuge using a single-use bag, thereby completing the present invention.

Accordingly, an object of the present invention is to provide a method for purifying a surface antigen protein from an influenza virus using a detergent, in which a detergent treatment concentration for treatment of a sampled influenza virus is determined by a value obtained by determining a total protein quantification value (TPQV) per unit dose of the sampled influenza virus and then substituting the total protein quantification value into the following Equation 1:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose (volume) of the sampled influenza virus to be treated with a detergent).

Another object of the present invention is to provide a test method for rapid confirmation of influenza antigen purification conditions, the method comprising a first purification condition determination method and a second purification condition determination method:

the first purification condition determination method, which is used in a step of purifying an influenza virus from an influenza virus culture, and in which a condition for the purification of an influenza virus is determined based on a hemagglutination assay, SDS-PAGE, or a combination thereof for influenza virus samples obtained through purification under different conditions; and
the second purification condition determination method, which is used in a step of purifying a surface antigen protein from an influenza virus using a detergent, and in which a detergent treatment concentration during the purification of a surface antigen protein is determined based on a total protein quantification assay for an influenza virus sample.

Another object of the present invention is a vaccine manufacturing method comprising purifying a surface antigen protein from an influenza virus, in which the step is carried out under a condition of using a detergent for treatment in a value obtained by determining a total protein quantification value (TPQV) per unit dose of a sampled influenza virus and then substituting the total protein quantification value into the following Equation 1:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

Another object of the present invention is to provide a method for rapid purification of an influenza surface antigen, the method comprising the following steps:

a) infecting a cell with an influenza virus and culturing the cell to obtain a virus culture;
b) determining a purification condition based on a hemagglutination assay, SDS-PAGE, or a combination thereof for influenza virus samples obtained through purification under different conditions to purify the influenza virus from the culture of step a);
c) purifying the influenza virus from the culture of step a) according to the condition determined in step b);
d) determining a detergent treatment concentration during surface antigen protein purification based on a total protein quantification assay for an influenza virus to purify a surface antigen from the influenza virus purified in step c); and
e) purifying the surface antigen protein from the influenza virus by performing detergent treatment according to the condition determined in step d).

Another object of the present invention is a method for manufacturing a vaccine containing an influenza surface antigen, the method comprising the following steps, in which a vaccine manufacture period is shortened:

a) infecting a cell with an influenza virus and culturing the cell to obtain a virus culture;
b) determining a purification condition based on a hemagglutination assay, SDS-PAGE, or a combination thereof for influenza virus samples obtained through purification under different conditions to purify the influenza virus from the culture of step a);
c) purifying the influenza virus from the culture of step a) according to the condition determined in step b);
d) determining a detergent treatment concentration surface antigen protein purification based on a total protein quantification assay for an influenza virus to purify a surface antigen from the influenza virus purified in step c); and
e) purifying the surface antigen protein from the influenza virus by performing detergent treatment according to the condition determined in step d).

Another object of the present invention is to provide a method for producing an influenza virus, the method comprising:

(a) culturing a cell in a single-use bioreactor (SUB);
(b) exchanging a portion of the medium in the cell culture solution of step (a) for a fresh medium using a low-speed continuous centrifuge using a single-use bag;
(c) infecting the propagated cells with an influenza virus and culturing the cells under a condition permissive for replication of the influenza virus; and
(d) isolating the influenza virus from the culture of step (c).

Another object of the present invention is a method for manufacturing a vaccine containing an influenza antigen, the method comprising the following steps, in which a vaccine manufacture period is shortened:

(a) culturing a cell in a single-use bioreactor (SUB);
(b) exchanging a portion of the medium in the cell culture solution of step (a) for a fresh medium using a low-speed continuous centrifuge;
(c) infecting the propagated cells with an influenza virus and culturing the cells under a condition permissive for replication of the influenza virus; and
(d) isolating the influenza virus from the culture of step (c).

**[Technical Solution]**

In order to achieve the object of the present invention described above, the present invention provides a method for purifying a surface antigen protein from an influenza virus using a detergent, in which a detergent treatment concentration for treatment of a sampled influenza virus is determined by a value obtained by determining a total protein quantification value (TPQV) per unit dose of the sampled influenza virus and then substituting the total protein quantification value into the following Equation 1:

[Equation 1]

detergent treatment concentration = [{(a*TPQV(µg/mL))/(b µg/mL)}/c]*d

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

In order to achieve another object of the present invention, the present invention provides a test method for rapid confirmation of influenza antigen purification conditions, the method comprising a first purification condition determination method and a second purification condition determination method:

the first purification condition determination method, which is used in a step of purifying an influenza virus from an influenza virus culture, and in which a condition for the purification of an influenza virus is determined based on a hemagglutination assay, SDS-PAGE, or a combination thereof for influenza virus samples obtained through purification under different conditions; and
the second purification condition determination method, which is used in a step of purifying a surface antigen protein from an influenza virus using a detergent, and in which a detergent treatment concentration during the purification of a surface antigen protein is determined based on a total protein quantification assay for an influenza virus sample.

In order to achieve another object of the present invention, the present invention provides a vaccine manufacturing method comprising purifying a surface antigen protein from an influenza virus, in which the step is carried out under a condition of using a detergent for treatment in a value obtained by determining a total protein quantification value (TPQV) per unit dose of the sampled influenza virus and then substituting the total protein quantification value into the following Equation 1:

[Equation 1]

detergent treatment concentration = [{(a*TPQV(µg/mL))/(b µg/mL)}/c]*d

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

In order to achieve another object of the present invention, the present invention provides a method for rapid purification of an influenza surface antigen, the method comprising the following steps:

a) infecting a cell with an influenza virus and culturing the cell to obtain a virus culture;
b) determining a purification condition based on a hemagglutination assay, SDS-PAGE, or a combination thereof for influenza virus samples obtained through purification under different conditions to purify the influenza virus from the culture of step a);

c) purifying the influenza virus from the culture of step a) according to the condition determined in step b);

d) determining a detergent treatment concentration during surface antigen protein purification based on a total protein quantification assay for an influenza virus to purify a surface antigen from the influenza virus purified in step c); and

e) purifying the surface antigen protein from the influenza virus by performing detergent treatment according to the condition determined in step d).

In order to achieve another object of the present invention, the present invention provides a method for manufacturing a vaccine containing an influenza surface antigen, the method comprising the following steps, in which a vaccine manufacture period is shortened:

a) infecting a cell with an influenza virus and culturing the cell to obtain a virus culture;

b) determining a purification condition based on a hemagglutination assay, SDS-PAGE, or a combination thereof for influenza virus samples obtained through purification under different conditions to purify the influenza virus from the culture of step a);

c) purifying the influenza virus from the culture of step a) according to the condition determined in step b);

d) determining a detergent treatment concentration during surface antigen protein purification based on a total protein quantification assay for an influenza virus to purify a surface antigen from the influenza virus purified in step c); and

e) purifying the surface antigen protein from the influenza virus by performing detergent treatment according to the condition determined in step d).

In order to achieve another object of the present invention, the present invention provides a method for producing an influenza virus, the method comprising:

(a) culturing a cell in a single-use bioreactor (SUB);

(b) exchanging a portion of the medium in the cell culture solution of step (a) for a fresh medium using a low-speed continuous centrifuge using a single-use bag;

(c) infecting the propagated cells with an influenza virus and culturing the cells under a condition permissive for replication of the influenza virus; and

(d) isolating the influenza virus from the culture of step (c).

In order to achieve another object of the present invention, the present invention provides a method for manufacturing a vaccine containing an influenza antigen, the method comprising the following steps, in which a vaccine manufacture period is shortened:

(a) culturing a cell in a single-use bioreactor (SUB);

(b) exchanging a portion of the medium in the cell culture solution of step (a) for a fresh medium using a low-speed continuous centrifuge using a single-use bag;

(c) infecting the propagated cells with an influenza virus and culturing the cells under a condition permissive for replication of the influenza virus; and

(d) isolating the influenza virus from the culture of step (c).

Hereinafter, the present invention will be described in detail.

The present invention provides a method for purifying a surface antigen protein from an influenza virus using a detergent, in which a detergent treatment concentration for treatment of a sampled influenza virus is determined by a value obtained by determining a total protein quantification value (TPQV) per unit dose of the sampled influenza virus and then substituting the total protein quantification value into the following Equation 1:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),

b is 100, 200, 300, 400, 500, 600, 700, 800, or 900, and a value closest to the TPQV is selected,

c is a concentration of detergent stock used for treatment (% (v/v)), and

d is a dose of the sampled influenza virus to be treated with a detergent).

The virion structure of influenza virus has an envelope consisting of a lipid bilayer on the outermost side, from which two surface glycoproteins protrude, one is a columnar hemagglutinin (HA) and the other is a mushroom-shaped neuraminidase (NA).

The method provided by the present invention is a method for isolating and purifying the hemagglutinin and neuraminidase, which are surface antigens of influenza virus, by detergent treatment, in which the optimal treatment concentration of the detergent used to isolate the surface antigen of influenza virus from the viral core is quickly calculated and the influenza virus surface antigen purification conditions are established in a short period of time.

In the present invention, the "detergent" is used interchangeably with "surfactant", and the type of detergent is not limited as long as it is used to isolate and purify the surface antigen of a virus in the art, and may include, for example, non-ionic or ionic (for example, cationic) detergents. Non-limiting examples of the detergent include alkylglycosides, alkylthioglycosides, acyls, sugars, sulfobetaines, betaine, polyoxyethylene alkyl ether, *N,N*-dialkyl-glucamide, Hecameg, alkylphenoxy-polyethoxyethanol, quaternary ammonium compounds, sarcosyl, CTAB (cetyl trimethyl ammonium bromide), tri-*N*-butyl phosphate, Cetavlon, myristyltrimethylammonium salt, lipofectin, lipofectamine, and DOTMA, octyl- or nonyl-phenoxy polyoxyethanol (for example, Triton detergents such as Triton X-100 or Triton N101), polyoxyethylene sorbitan esters (Tween detergents), polyoxyethylene ethers, and polyoxyethylene esters.

In a preferred embodiment of the present invention, the detergent may be a cationic detergent.

In a more preferred embodiment of the present invention, the detergent may be CTAB.

The influenza virus of the present invention includes types A, B and C depending on the molecular biological characteristics. Preferably, the influenza virus may be type A or B. Among these, the influenza A virus may be classified into various subtypes depending on the types of hemagglutinin (HA) and neuraminidase (NA) glycoproteins, which are surface antigens. More specifically, since 18 types of hemagglutinin and 11 types of neuraminidase are known as surface antigens of influenza A virus, arithmetically there may be a total of 198 subtypes of influenza A virus, all of which may be understood to be included in the influenza virus of the present invention.

Meanwhile, influenza viruses of new subtypes to be revealed in the future as well as subtypes known to date, including antigenic shift in which the HA and NA, which are the main antigens, are changed to new subtypes completely different from those previously known, or antigenic drift in which a small number of amino acids are changed due to accumulation of point mutations at the HA and NA gene levels, may be included in the influenza virus of the present invention without limitation.

In a preferred embodiment of the present invention, the influenza virus may be influenza A virus, and non-limiting examples thereof include H1N1, H2N2, H3N2, H5N1, H7N7, H1N2, H9N2, H7N2, H7N3, H10N7, H7N9, H6N1, and the like.

In the present invention, the "sampled" influenza virus may mean an influenza virus subjected to one or more steps selected from the group consisting of a step of cultivating an influenza virus expressing a surface antigen protein in a host cell to provide a culture, a step of purifying and/or concentrating the culture, and a step of inactivating the influenza virus contained in the culture.

In an embodiment of the present invention, the sampling may mean that the influenza virus is treated by a method comprising the following steps:

(i) providing a cell-based virus propagation culture solution or fertilized egg-based virus propagation culture solution in which an influenza virus can be propagated;

(ii) clarifying the culture solution to obtain a culture containing the influenza virus;

(iii) optionally purifying and optionally concentrating the culture; and

(iv) optionally inactivating the influenza virus contained in the culture.

In step (i), a culture solution containing an influenza virus is provided. Such a culture solution may be derived from a cell-based viral propagation culture solution or a fertilized egg-based viral propagation culture solution. As is known to those skilled in the art, viruses may be propagated in cell-based culture systems or in fertilized egg-based culture systems. Suitable cell lines used to grow influenza viruses are typically of mammalian origin, and thus represent mammalian cell lines, preferably animal cell lines. Suitable animal cell lines are known to those skilled in the art and may include cell lines of hamster, bovine, primate (including human and monkey) and canine origin. As a non-limiting example of the cell line, cells of the mammalian cell lines may include Vero, PerC6, BHK, 293, COS, PCK, MRC-5, MDCK, MDBK, WI-38, and serum-free adaptive cells thereof. Preferably, the cell line may be MDCK cells.

The influenza virus may be grown on cells by any suitable method known to those skilled in the art. For cell-based culture systems, any suitable medium may be used. In a preferred embodiment, the cells may be cultured in serum-free and/or protein-free media. For cell-based viral propagation, generally viruses or live virus preparations are each inoculated into the cell culture in which they are propagated. Virus propagation may be performed in a batch mode, or in a fed-batch mode, or in a perfusion mode.

Alternatively, the influenza virus may be propagated in fertilized egg-based systems. In fertilized egg-based manufacture, generally a working seed virus is injected into embryonated eggs of laying hens. The virus starts to propagate in the allantoic fluid of the embryonated egg, and the amount of virus is increased. For example, after 2 to 3 days of incubation, the embryonated eggs are cooled to 2°C to 8°C, and the allantoic fluid of each embryonated egg is harvested, and pooled using a manual or automated vacuum harvesting system. The collected virus-containing allantoic fluid is a fertilized egg-based culture solution containing viruses and is further processed in a subsequent purification process.

Depending on the culture system used to propagate the influenza virus, the culture solution containing propagated viral particles may contain different contaminants. For example, potential contaminants present in fertilized egg-based culture solutions are fertilized egg-based proteins, for example, fertilized egg albumin, or antibiotics, but typical contaminants present in cell-based culture solutions are residual host cell DNA. Preferably, in the methods provided by the present invention, the system used to propagate the influenza virus is not limited; and those obtained by treating the culture solutions containing the viruses propagated in the cell-based culture system and similarly in the fertilized egg-based culture system may all be applied.

In the present invention, the culture solution of step (i) may be manufactured by using a single-use bioreactor. By using such a single-use bioreactor, for example, the obtained product itself may be improved, for example, in terms of quality and/or purity. This may be particularly advantageous when the method is carried out on a large scale, for example, on an industrial scale.

The cell-based or fertilized egg-based culture solution containing influenza viruses may be clarified (step (ii)). The clarification step is performed on culture solutions containing viral particles, but not on sedimented or otherwise pelletized substances. In the present invention, the purpose of such a clarification step is to remove particulate substances, for example, cell debris, protein impurities and/or support substances used to grow cells, for example, microcarriers from the culture solution. The protein impurities may be, for example, allantoin, collagen and/or albumin, for example, ovalbumin. For clarification, any suitable means or method known to those skilled in the art may be used. In particular, tangential flow filtration (TFF), depth filtration, or centrifugation may be applied. Therefore, the respective settings of the respective applied clarification means (TFF, depth filtration, centrifugation) are selected in such a way that the purpose indicated above is achieved. In general, when such a purpose is given, each setting for each means is known to those skilled in the art.

In the present invention, the clarified culture solution obtained by the method may be referred to as a "virus culture".

In an aspect of the present invention, the virus culture may be the "sampled influenza virus" provided in the present invention.

In another aspect of the present invention, the "sampled influenza virus" may be one additionally subjected to the following step (iii) and/or step (iv) after step (ii).

In an aspect of the present invention, purification of the culture in step (iii) may be performed by any suitable method for specifically purifying the virus antigen. The preferred purification method includes, for example, chromatography, for example, ion exchange chromatography, hydrophobic interaction chromatography, pseudo-affinity chromatography, affinity chromatography, size exclusion chromatography, liquid-liquid chromatography and the like. In an embodiment, the preferred purification method is affinity chromatography, and a more preferred purification method is affinity chromatography using Cellufine Sulfate (CS) as a carrier.

In an aspect of the present invention, when CS affinity chromatography is used to purify the culture in step (iii), the selection of column dimensions may be determined by those skilled in the art. In the CS affinity chromatography, the CS column may be equilibrated using an equilibration buffer to load the influenza virus filtrate. In an embodiment of the present invention, the CS column is equilibrated using an equilibration buffer, and the equilibration buffer may contain 10 mM to 20 mM sodium phosphate and may have a pH of 7.0 to 7.6.

In the purification process, the influenza virus attached to the CS column may be eluted with an elution buffer. In an embodiment of the present invention, the influenza virus attached to the CS column may be eluted when an elution buffer containing sodium chloride at a concentration of 0.01 M to 1.0 M is injected into the column at a neutral pH. The elution buffer may have a pH of 6.5 to 7.4, and may contain 0.1 M to 6.0 M sodium chloride.

In order to wash away any protein bound to the CS column during the purification process, the CS column may be washed using an elution buffer containing 1.0 M to 3.0 M sodium chloride. The used CS column may optionally be washed, stored in an appropriate agent, and optionally reused.

In an aspect of the present invention, the concentration in step (iii) may be achieved by a suitable method. The method leads to a reduction in the volume of the virus culture. The concentration step aims to obtain a concentrated culture by reducing the volume of the culture containing influenza viruses.

In a preferred aspect, the volume may be reduced by applying TFF ultrafiltration or TFF ultrafiltration and diafiltration (TFF ultrafiltration/diafiltration) in a single step, and ultracentrifugation or precipitation, preferably TFF ultrafiltration may be used to reduce the volume. Therefore, the respective settings of the respective applied concentration means (TFF, TFF ultrafiltration/diafiltration, ultracentrifugation or precipitation) are selected in such a way that the purposes indicated above, namely volume reduction and concentration of influenza virus are achieved.

In another embodiment, when the volume is reduced by ultracentrifugation, the conditions of ultracentrifugation may be, for example, for longer than 10 minutes at 30,000 $g$ to for longer than 10 minutes at 200,000 $g$.

In another embodiment, when concentration is performed by applying precipitation, suitable chemicals are known to those skilled in the art and may be, for example, salts, methyl and ethyl alcohols and polyethylene glycol at appropriate concentrations, respectively. A suitable chemical at an appropriate concentration precipitates the product, for example, particles containing a virus antigen, but has minimal or no adverse effect on the virus antigen. The chemical is selected so as not to react with the desired antigen, for example, influenza virus surface antigen protein, and not to alter the immunogenicity of the desired antigen.

In an embodiment, the volume of the virus culture in the concentration step may be reduced by 4-fold or more, preferably 5-fold or more, or 9-fold or more, and preferably 12-fold or more, and preferably 13-fold or more, or 15-fold or more, and preferably 20-fold or more, or 25-fold or more.

In an aspect of the present invention, the virus purified and/or concentrated virus culture after step (iii) may be inactivated. The inactivation results in the elimination of infectivity of the virus and may typically be performed by treating the virus with an effective amount of one or more of the following chemical means: a detergent, formaldehyde, β-propiolactone, methylene blue, psoralen, carboxyfullerene (C60), diethylamine, acetyl ethylenimine, or a combination thereof. The inactivation method is known to those skilled in the art and includes, for example, treatment of the virus with physical means, for example, gamma radiation and/or UV light.

In an embodiment of the present invention, the inactivation by applying the same detergent as that used to isolate the influenza virus surface antigen may not be performed.

In another aspect of the present invention, the sampled influenza virus may mean one obtained by a method comprising the following steps:

   (a) culturing a cell in a single-use bioreactor (SUB);
   (b) exchanging a portion of the medium in the cell culture solution of step (a) for a fresh medium using a low-speed continuous centrifuge using a single-use bag;
   (c) infecting the propagated cells with an influenza virus and culturing the cells under a condition permissive for replication of the influenza virus; and
   (d) isolating the influenza virus from the culture of step (c).

Steps (a) to (d) will be described in more detail as follows:

   (a) culturing a cell in a single-use bioreactor (SUB);

In the present invention, step (a) is a step of culturing a host cell for propagating a virus through infection, in which cells are cultured in a single-use bioreactor. In an aspect, in step (a), a bioreactor system comprising a single-use member such as a flexible plastic bag for culturing cells is used. Such bioreactor systems are known in the art and are commercially available. For example, see International Patent Publications WO 05/108546, WO 05/104706, and WO 05/10849 and the following section 8.12. A bioreactor system comprising a single-use member (referred to herein as a "single-use bioreactor" or abbreviation "SUB") may be sterilized in advance; and there is no need for steam-in-place (SIP) or clean-in-place(CIP) for batch-to-batch or product-to-product conversion in the culture or production systems. SUB rarely requires regulatory control since it can be confirmed that there is no batch-to-batch contamination, and can be rapidly disposed with minimal preparation or no separate preparation before use, facilitating the production of large quantities of vaccine ingredients from cell cultures. SUB can thus be operated with a significant advantage in terms of cost and time. In another aspect, the single-use bioreactor system may be a stirred-tank reactor system capable of providing a hydrodynamic environment for mixing of a cell culture with more efficient adjustment of nutrients, $O_2$ and pH.

In an aspect of the present invention, as the cell, any cell capable of achieving the purpose of virus propagation by being infected with a virus and cultured may be used in the present invention without limitation. In particular, suitable cells used to grow influenza viruses may typically be of mammalian origin. Suitable animal cells are known to those skilled in the art and may include cell lines of hamster, bovine, primate (including human and monkey) and canine origin. As a non-limiting example of the cell line, mammalian cells may include Vero, PerC6, BHK, 293, COS, PCK, MRC-5, MDCK, MDBK, WI-38, and serum-free adaptive cells thereof. Preferably, the cell line may be MDCK cells.

As the MDCK cells, for example, those derived from ATCC CCL-34 cell line, ATCC PTA-7909 or PTA-7910 may be used. In an exemplary embodiment, the MDCK Sky1023 (DSM ACC3112), MDCK Sky10234 (DSM ACC3114), or MDCK Sky3851 (DSM ACC3113) cell line derived from ATCC CCL-34 disclosed in Korean Patent Publication No. 10-2012-0024464 or an MDCK cell line such as the MDCKS-MG cell line (KCLRF-BP-00297) disclosed in Korean Patent No. 10-1370512 may be used. Particularly preferably, the MDCK Sky3851 cells may be used, which do not require serum for cell growth, can be cultured in suspension without the need for a separate carrier for attachment, and have low tumorigenicity.

In a preferred embodiment, the medium for culturing cells may be a serum-free culture medium. Since animal sera may contain other pathogenic substances, which have a potential risk of transmission to humans or animals being treated or vaccinated with vaccines manufactured from cell cultures, and may be fatal, especially in immunocompromised humans. As the serum-free culture medium, for example, well-known cell culture media such as MEM media such as Eagle's minimal essential media (EMEM) and Dulbecco's modified Eagle's medium (DMEM) may be used, or those derived from these cell culture media or modified from these cell culture media may be used. For example, a commercially available culture medium such as VP-SFM manufactured by Gibco BRL/Life Technology may be used. Culture media derived from these culture media may be used to induce subculture of cells.

The medium may contain, for example, a plant hydrolysate obtained from one or more of corn, pea, soybean, malt, potato, or wheat; lipid supplements such as cholesterol or saturated and/or unsaturated fatty acids; trace elements such as $CuSO_4 \cdot 5H_2O$, $ZnSO_4 \cdot 7H_2O$, and iron citrate; one or more hormones such as growth factors; putrescine, amino acids, vitamins, fatty acids such as unsaturated fatty acids, nucleosides, sodium bicarbonate, carbon sources such as glucose, iron binding substances, and the like. The content of these components that can be added to the cell culture medium is well known in the art.

In an aspect of the present invention, the cells may be cultured at a $CO_2$ concentration of 1% or more, or 2% or more, 3% or more, or 4% or more, or 5% or more, or 6% or more, or 7% or more, or 8% or more, or 9% or more, or 10% or more, or 20% or more.

In an aspect of the invention, the dissolved oxygen (DO) concentration ($pO_2$ value) in the single-use bioreactor is advantageously adjusted upon culturing the cells and ranges from 5% to 95% (based on air saturation), or from 10% to 60%. In a certain embodiment, the dissolved oxygen (DO) concentration ($pO_2$ value) may be 10% or more, or 20% or more, or 30% or more, or 50% or more.

In an aspect of the present invention, the pH of the culture medium used for culturing cells is adjusted during culture, and may be in the range of pH 6.4 to pH 8.0, or the range of pH 6.8 to pH 7.6. In a certain embodiment, the pH of the culture medium may be 6.4 or more, or 6.6 or more, or 6.8 or more, or 7.0 or more, or 7.2 or more.

In an aspect of the present invention, the cells may be cultured at a temperature of 25°C to 39°C. In a certain embodiment, the culture temperature may be 30°C to 38°C, or 35°C to 38°C, or 36°C to 38°C.

In an aspect of the present invention, the cells may be cultured while one or more parameters selected from the group consisting of temperature, stirring speed, pH, dissolved oxygen (DO), flow rates of $O_2$ and $CO_2$ are monitored and/or controlled in the stirred-tank SUB. In an embodiment, the temperature is maintained at about 30°C to about 42°C, or about 33°C to about 39°C, or about 35°C to about 38°C. In a certain embodiment, the temperature is maintained at about 36°C to about 37°C. In an embodiment, the stirring speed is maintained at about 50 rpm to 150 rpm. In a specific embodiment, the stirring speed is maintained at about 60 rpm to about 120 rpm, or about 70 rpm to about 100 rpm. The stirring speed is adjusted by methods well known in the art. In another embodiment, the pH of the culture is maintained at about 6.0 to about 7.5. In a specific embodiment, the pH at the start of culture is about 6.0 to about 7.5, and the pH of the culture is maintained at about 7.0 to about 7.5 during culture. The starting pH may be lower or higher than the above preferred range, and it is also possible for those skilled in the art to raise or lower this pH to a desired level (for example, 7.4) and maintain the pH at that level. Such a pH is maintained by any method known in the art. For example, the pH may be adjusted by sparging of $CO_2$ and/or addition of an acid (for example, HCl) or a base (for example, NaOH) as needed. In yet another embodiment, the acceptable DO range is about 100% to about 35%. In a certain embodiment, DO is maintained at about 35% to about 70%, or about 50%. In another specific embodiment, DO is required not to drop to less than about 35%. Initial DO may be 100%, and it is also possible for those skilled in the art to drop this DO to a preset concentration (for example, 50%) and maintain DO at this level. DO may be maintained by any method known in the art, for example, by sparging of $O_2$.

(b) exchanging a portion of the medium in the cell culture solution of step (a) for a fresh medium using a low-speed continuous centrifuge using a single-use bag;

Step (b) is a step of exchanging the medium before the cells cultured in step (a) are sufficiently propagated and infected with a virus.

In an aspect of the present invention, the medium exchange of step (b) is performed by a method in which the cells and the medium are continuously separated from each other using a low-speed continuous centrifuge using a single-use bag without a process of recovering and reintroducing the culture solution, a certain amount of the medium is discarded, and a new medium is introduced again into the single-use bag. Unlike a general method in which the entire amount of the culture solution is recovered and then divided into smaller portions to be put in containers and centrifugation is performed, when a low-speed continuous centrifuge using a single-use bag is used, medium exchange can be performed in a closed system and the possibility of contamination can be greatly diminished.

In an aspect of the present invention, the "portion of the medium" may be 50% to 90% of the volume of the entire culture solution present in the single-use bag. The portion of the medium may be 60% to 80%, most preferably 70% to 80% of the volume of the entire culture solution present in the single-use bag.

In an embodiment, the medium is exchanged for a medium having the same volume and the same composition. Specifically, when the cells are precipitated using a low-speed continuous centrifuge, a portion of the upper medium is recovered, and a medium having the same volume as that of the recovered medium is supplemented to perform medium exchange. In another embodiment, the medium is exchanged for a medium having the reduced volume to effectively concentrate the cells. The medium may be exchanged for a medium having the same or different composition. In an embodiment, the growth medium used for propagation of the cells in step (a) may be exchanged for the infection medium (namely, medium used for infection and virus replication) used in step (c) below. Optionally, the growth medium is supplemented with and/or contains additional components (for example, glucose, trace minerals, amino acids, and the like) so that medium exchange is not required. In another certain embodiment, the infection medium contains a serine protease (for example, trypsin, TrypLE, or the like). In another embodiment, where the medium has not been changed, a serine protease (for example, trypsin, TrypLE, or the like) is added immediately before, during, or immediately after infection. In a certain embodiment, a protease is added prior to or concurrently with the infection of cells with a virus.

(c) infecting the propagated cells with an influenza virus and culturing the cells under a condition permissive for replication of the influenza virus;

In an embodiment, the influenza virus may be administered to the culture medium in a dosage to have a multiplicity of infection (MOI) of approximately 0.00001 to 0.01, preferably an MOI of 0.0001 to 0.002. With such a dosage, the cells inoculated into the culture medium may be sufficiently infected, and propagation of the influenza virus in the desired amount may be expected.

In an embodiment, after infection, the cells are cultured at a temperature of 22°C to 40°C. In a certain embodiment, after being infected with the virus, the cells are cultured at a temperature of 39°C or less, or 38°C or less, or 37°C or less, or 36°C or less, or 35°C or less, or 34°C or less, or 33°C or less, or 32°C or less, or 30°C or less, or 28°C or less, or 26°C or less, or 24°C or less. In a certain embodiment, after being infected with the virus, the cells are cultured at a temperature of 33°C. In another embodiment, after being infected with the virus, the cells are cultured at a temperature of 33°C or less. In yet another embodiment, after infection, the cells are cultured at a temperature of 31 °C. In a certain embodiment, culture of the cells is performed for 2 to 10 days. Culture may be performed by a batch process.

In an aspect of the present invention, culture in step (c) is performed after a period sufficient to produce the virus in an appropriate yield, for example, 2 to 10 days after infection, or optionally 3 to 7 days after infection. In an aspect of the present invention, culture in step (c) is performed 2 days, or 3 days, or 4 days, or 5 days, or 6 days, or 7 days, or 8 days, or 9 days, or 10 days after infection.

(d) isolating the influenza virus from the culture of step (c);

Step (d) is a step of purifying and isolating the influenza virus from the virus culture obtained in step (c).

As for the method of purifying the influenza virus in step (d), the above description may be equally applied.

In an aspect of the present invention, step (d) may be performed by a method comprising (d-1) determining a purification condition based on a hemagglutination assay for influenza virus samples obtained through purification under different conditions to purify the influenza virus from the culture of step c); and (d-2) purifying the influenza virus from the culture of step (c) according to the condition determined in step (d-1).

In step (d-1), the influenza virus culture is applied to the chromatography column, and then the column flowthroughs passed through the column under different conditions are each subjected to the hemagglutination assay to select a condition having the lowest hemagglutinin (HA) titer. As a result of the hemagglutination assay, it is not preferable that the HA titer of the virus culture passed through the column is high since this is a condition under which the virus in the virus culture cannot bind to the column but passes through the column as it is.

In another aspect of the present invention, in step (d-1), the influenza virus culture is applied to the chromatography column under different conditions, then the influenza virus bound to the column is eluted using a buffer solution, and the eluate according to each condition is subjected to the hemagglutination assay, SDS-PAGE, or a combination thereof to select a condition under which the HA titer is highest and HA is well observed on SDS-PAGE.

In another embodiment of the present invention, step (d-1) may be a combination of a condition under which the virus binds to the resin most favorably when the virus culture passes through the column and a condition under which the virus can be eluted most favorably in the process of eluting the virus bound to the resin. The conditions under which the virus binds to the resin most favorably may be selected by subjecting the column flowthrough passed through the column to the hemagglutination assay, SDS-PAGE, or a combination thereof, and the condition under which the virus can be eluted most favorably in the process of eluting the virus bound to the resin may be selected by subjecting the eluate passed through the column to the hemagglutination assay, SDS-PAGE, or a combination thereof.

In another aspect of the present invention, in step (d-1), the virus cultures passed through the chromatography column under different conditions may be subjected to the hemagglutination assay and SDS-PAGE to select a condition under which the influenza virus is least lost.

In an aspect of the present invention, a step of concentrating the purified virus culture may be additionally performed after step (d). As for the method of concentrating the purified virus culture, the above description may be equally applied.

Thereafter, the detergent treatment concentration for treatment of the sampled influenza virus may be determined by a value obtained by determining a total protein quantification value (TPQV) per unit dose of the influenza virus culture sampled by the method described above and then substituting the total protein quantification value into the following Equation 1 :

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

The virus surface antigen vaccine is a high-quality vaccine with few side effects and high purity, and it is greatly important to set an appropriate detergent treatment concentration. When an excessive amount of detergent is used for treatment, the whole virus is destroyed, and the protein inside the viral core, which is an impurity, is exposed to the outside, resulting in a decrease in purity. When the detergent treatment concentration is insufficient, most surface antigens are not solubilized and the yield of surface antigens decreases.

Therefore, in an aspect of the present invention, the detergent treatment concentration calculated according to the equation is a concentration for selectively solubilizing only the surface antigen of influenza virus in a state in which the viral core is not destroyed or a state in which destruction of the viral core is minimized.

Meanwhile, it is an important factor in the influenza vaccine production process to quickly confirm an appropriate detergent treatment concentration for solubilization of the virus surface antigen. Currently, single radial immunodiffusion (SRID) recommended by the European Pharmacopoeia and the World Health Organization (WHO) is commonly used at home and abroad to measure the surface antigen content of influenza vaccines, but SRID has a limitation in that the time required to acquire a result is too long.

Therefore, the present invention provides a method in which the treatment concentration of the detergent added is determined based on the total protein quantification value per unit dose of the virus culture, but the quantification process by SRID for the amount of hemagglutinin (HA) protein in the virus culture is not required.

The total protein quantification value (TPQV) per unit dose of the virus culture in the method of the present invention may be determined by any method used for calculating a protein content in the art, and non-limiting examples thereof may include a BCA assay, a Lowry assay, or a Bradford assay.

In Equation 1 of the present invention, the a-value means the concentration (%) of the detergent used for treatment based on the protein content. Since the optimal concentration of detergent used for treatment may be different depending on the strain of the virus and the properties of the virus, it is possible to confirm the appropriate concentration (%) of detergent used for treatment depending on the virus by performing SDS-PAGE for any value within the range of 0.005% (v/v) to 0.100% (v/v). Specifically, the virus solution is divided into small portions and treated with a detergent in any value within the numerical range, and then the optimal concentration of detergent used for treatment is confirmed in which the surface antigen protein is recovered as a supernatant and the viral core protein is isolated as a precipitate by performing ultracentrifugation, whereby the optimal concentration of detergent used for treatment may be determined.

In the present invention, the b-value is 100, 200, 300, 400, 500, 600, 700, 800, or 900, and a value closest to the TPQV is selected. For example, if the TPQV of the sampled influenza virus is 220 $\mu$g/mL, 200, which is the closest value among the values, may be the b-value, and if the TPQV is 370 $\mu$g/mL, 400, which is the closest value among the values, may be the b-value.

In the present invention, the TPQV may vary depending on the concentration degree of the sampled influenza virus. Therefore, in order to determine the optimal detergent treatment concentration according to Equation 1 of the present invention, it is preferable to use a concentrated influenza virus culture so that the sampled influenza virus has a TPQV of 50 $\mu$g/mL to 950 $\mu$g/mL.

The present invention also provides a test method for rapid confirmation of influenza antigen purification conditions, the method comprising a first purification condition determination method and a second purification condition determination method:

the first purification condition determination method, which is used in a step of purifying an influenza virus from an influenza virus culture, and in which a condition for the purification of an influenza virus is determined based on a hemagglutination assay, SDS-PAGE, or a combination thereof for influenza virus samples obtained through purification under different conditions; and

the second purification condition determination method, which is used in a step of purifying a surface antigen protein from an influenza virus using a detergent, and in which a detergent treatment concentration during the purification of a surface antigen protein is determined based on a total protein quantification assay for an influenza virus sample.

In an embodiment of the present invention, the first purification corresponds to the virus culture purification step of step (c) described above.

In another aspect of the present invention, the first purification does not require a quantification process by SRID (single radial immunodiffusion) for the amount of hemagglutinin (HA) protein in the virus culture.

In another aspect of the present invention, the step of purifying the influenza virus from the influenza virus culture may be performed by chromatography.

In another aspect of the present invention, the chromatography includes, for example, ion exchange chromatography, hydrophobic interaction chromatography, pseudo-affinity chromatography, affinity chromatography, size exclusion chromatography, liquid-liquid chromatography and the like. In an embodiment, the preferred purification method is affinity chromatography, and a more preferred purification method is affinity chromatography using Cellufine Sulfate (CS) as a carrier.

In another aspect of the present invention, in the first purification, the condition under which the influenza virus culture passed through the column can bind to the resin most favorably may be confirmed and then the elution condition under which the bound virus can be isolated and purified with optimal yield and purity may also be selected.

In another aspect of the present invention, in the first purification condition determination method, the influenza virus culture may be diluted with an equilibration buffer by applying various ratios and applied to a chromatography column, and then the column flowthroughs passed through the column under different conditions may each be subjected to the hemagglutination assay to select a condition having the lowest hemagglutinin (HA) titer. As a result of the hemagglutination assay, it is not preferable that the HA titer of the column flowthrough passed through the column is high since this is a condition under which the virus in the virus culture cannot bind to the column but passes through the column as it is.

In another aspect of the present invention, in the first purification condition determination method, the influenza virus culture may be diluted with an equilibration buffer by applying various ratios and applied to a chromatography column under different conditions, then the influenza virus bound to the column may be eluted using an elution buffer, and the eluate according to each condition may be subjected to the hemagglutination assay, SDS-PAGE, or a combination thereof to select a condition having the highest HA titer and the thickest hemagglutinin band on SDS-PAGE.

In another aspect of the present invention, the first purification condition may be a combination of a condition under which the virus binds to the resin most favorably when the virus culture passes through the column and a condition under which the virus can be eluted most favorably in the process of eluting the virus bound to the resin. The condition under which the virus binds to the resin most favorably may be selected by subjecting the column flowthrough passed through the column to the hemagglutination assay, SDS-PAGE, or a combination thereof, and the condition under which the virus can be eluted most favorably in the process of eluting the virus bound to the resin may be selected by subjecting the eluate passed through the column to the hemagglutination assay, SDS-PAGE, or a combination thereof.

In another aspect of the present invention, in the first purification condition determination method, the virus cultures passed through the chromatography column (namely, column flowthroughs passed through the column) under different conditions may be subjected to the hemagglutination assay, SDS-PAGE, or a combination thereof to select a condition under which the influenza virus is least lost.

In another embodiment of the present invention, the conditions may be one or more selected from the group consisting of the type of buffer, the concentration of the buffer, the pH and conductivity of the buffer. Preferably, the conditions may be the pH or conductivity of the buffer.

In an embodiment of the present invention, the protein stability was increased by maintaining the pH of the buffer at 7.0 to 7.4, and the conductivity was lowered to search for the conditions under which the virus binds to the resin most favorably. The conductivity of the influenza virus culture secured in the influenza virus cultivation process was about 10.0 mS/cm to 15.0 mS/cm, and the influenza virus culture was diluted with a sodium phosphate buffer having a low conductivity to confirm the binding capacity while lowering the conductivity of the sample to be loaded on the column. In order to rapidly confirm the purification conditions, the time and variables required to confirm the purification conditions were diminished by adjusting only the conductivity, except for the pH, which is set as one of the purification conditions.

In general, it is known that the influenza virus binds to the resin more favorably as the conductivity is lower, but the volume of the sodium phosphate buffer and the time for the chromatography process increase when the conductivity is lowered, this increases the production period and production cost, and it is thus important to confirm the proper conductivity. Therefore, in an embodiment of the present invention, while lowering the conductivity to 10.0 mS/cm or less in a manner of diluting the virus culture solution 0.5 to 4 times using a sodium phosphate buffer, the hemagglutination assay was performed on the virus culture passing through the column to confirm whether the binding capacity was proper. When the hemagglutination assay was performed, the test results could be acquired within 1 hour, and thus the purification results according to each condition were acquired within 1 day.

In another embodiment of the present invention, the elution of the influenza virus bound to the resin may be performed in a manner that searches for the optimal conductivity using a buffer solution at an appropriate concentration at which binding between the resin and the influenza virus can be cleaved.

According to an embodiment of the present invention, as a result of adding the buffer to the column while increasing the salt concentration under the 2 M to 5 M sodium chloride buffer/linear gradient condition, a peak appeared at UV280 while the virus, protein, and the like were eluted, and the fractions were recovered and analyzed to confirm the conductivity of and salt concentration in the fraction containing the virus. The virus was eluted under step gradient conditions using the 2 M to 5 M sodium chloride buffer and an equilibration buffer to confirm whether most influenza viruses were eluted at the corresponding elution concentration, and then the virus elution condition was established. The hemagglutination assay and/or SDS-PAGE were performed to confirm whether the virus was eluted with high yield and high purity.

In an aspect of the present invention, in the second purification condition determination method, the detergent treatment concentration for treatment of the virus solution may be determined by a value obtained by determining a total protein quantification value (TPQV) per unit dose of the influenza virus culture obtained through the first purification and then substituting the total protein quantification value into the following Equation 1:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the influenza virus culture to be treated with a detergent).

The present invention also provides a vaccine manufacturing method comprising purifying a surface antigen protein from an influenza virus, in which the step is carried out under a condition of using a detergent for treatment in a value obtained by determining a total protein quantification value (TPQV) per unit dose of the sampled influenza virus and then substituting the total protein quantification value into the following Equation 1:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,

c is a concentration of detergent stock used for treatment (% (v/v)), and

d is a dose of a virus solution to be treated with a detergent).

The present invention also provides a method for rapid purification of an influenza surface antigen, the method comprising the following steps:

a) infecting a cell with an influenza virus and culturing the cell to obtain a virus culture;
b) determining a purification condition based on a hemagglutination assay, SDS-PAGE, or a combination thereof for influenza virus samples obtained through purification under different conditions to purify the influenza virus from the culture of step a);
c) purifying the influenza virus from the culture of step a) according to the condition determined in step b);
d) determining a detergent treatment concentration during surface antigen protein purification based on a total protein quantification assay for an influenza virus solution to purify a surface antigen from the influenza virus solution purified in step c); and
e) purifying the surface antigen protein from the influenza virus by treating the virus solution with a detergent according to the condition determined in step d).

In an aspect of the present invention, the method for rapid purification of an influenza surface antigen may further comprise removing cells and cell debris from the virus culture prior to step b).

In another aspect of the present invention, the removal of cells and cell debris from the virus culture may be performed by one or more methods selected from the group consisting of filtration, dialysis and centrifugation

In another aspect of the present invention, in step b), the purification condition may be determined based on the hemagglutination assay and SDS-PAGE.

In another aspect of the present invention, the method for rapid purification of an influenza surface antigen may comprise an additional purification process by one or more methods selected from the group consisting of ultrafiltration and diafiltration after step c).

In another aspect of the present invention, in step e), the surface antigen protein may be isolated by performing detergent treatment, then performing centrifugation (preferably, ultracentrifugation), and recover the supernatant. The surface antigen protein isolated from the viral core by detergent treatment may be efficiently isolated by centrifugation. Such isolation may be performed by pelleting the viral core, for example by ultracentrifugation. Virus surface proteins, for example, hemagglutinin and/or neuraminidase may be present in the supernatant and the viral core may be present in the pellet. The centrifugation conditions selected for pelleting the viral core are known to those skilled in the art. For example, ultracentrifugation may be either batch mode or continuous mode, and may be performed at a suitable temperature, for example 2°C to 25°C, more preferably 2°C to 8°C for longer than 10 minutes at 30,000 $g$ to for longer than 10 minutes at 200,000 $g$.

In another aspect of the present invention, the method for rapid purification of an influenza surface antigen may further comprise a detergent removing process after step e). Such removal of the detergent may be performed by any method suitable for this purpose, for example, by treatment with Amberlite or TFF. In the case of using treatment with Amberlite, Amberlite is added to the supernatant collected after step e) has been performed, for example, after the viral core was pelleted at a mass ratio of Amberlite to the cationic detergent of about 1:1 to 100:1, incubation is carried out at a suitable temperature, for example, a temperature in the range of 2°C to 8°C, for a suitable time, for example, 8 to 24 hours, suitably 16 hours. Subsequently, Amberlite may be removed by any suitable method known to those skilled in the art, for example, by using conventional flow filtration/dead end filtration, sieve, or TFF. In the case of using a filter, the filter is preferably selected in such a way that the filter can retain particles larger than 5 $\mu$m or larger than 7 $\mu$m. Amberlite may also be removed by using a sieve. A suitable sieve opening may be in the range of 0.1 $\mu$m to 200 $\mu$m, preferably less than 150 $\mu$m, and more preferably less than 100 $\mu$m.

According to an embodiment of the present invention, CTAB used as a detergent was removed by adding Amberlite XAD-4 (macroreticular cross-linked aromatic polymer). CTAB was removed by direct adsorption because of its high molecular weight and ionicity. The ultracentrifugation supernatant was treated with Amberlite XAD-4 to adsorb CTAB, the reaction was conducted with stirring for 3 hours or more under a refrigeration condition of 2°C to 8°C, and then Amberlite XAD-4 was removed by filtration through a 0.22 $\mu$m filter.

In another aspect of the present invention, the method for rapid purification of an influenza surface antigen may comprise an additional chromatography performing process for removal of impurities such as DNA after step e). The chromatography may include, for example, ion exchange chromatography, hydrophobic interaction chromatography, pseudo-affinity chromatography, affinity chromatography, size exclusion chromatography, liquid-liquid chromatography and the like, and may be preferably anion exchange chromatography, most preferably TMAE (trimethyl aminoethyl) anion exchange chromatography. DNA is an anion, and can be easily isolated and removed using the property of binding to anion exchange resin.

In another aspect of the present invention, the TMAE column may be equilibrated using a sodium phosphate buffer for equilibration, and the equilibration buffer may contain 1 mM to 20 mM sodium phosphate and have a pH of 6.8 to 7.6.

In an embodiment of the present invention, in order to bind DNA to resin and recover the influenza surface antigen as a column flowthrough, the conductivity was adjusted by adding a sodium phosphate buffer having a pH of 6.5 to 7.4 and containing 1 M to 6 M sodium chloride to the influenza surface antigen process solution subjected to the Amberlite XAD-4 adsorption filtration process, and a proper level of surface antigen recovery was confirmed at a conductivity of 30 mS/cm to 120 mS/cm.

In another aspect of the present invention, the method for rapid purification of an influenza surface antigen may further comprise an additional purification process by one or more methods selected from the group consisting of ultrafiltration and diafiltration after the chromatography for removal of impurities.

The present invention also provides a method for manufacturing a vaccine containing an influenza surface antigen, the method comprising the following steps, in which a vaccine manufacture period is shortened:

a) infecting a cell with an influenza virus and culturing the cell to obtain a virus culture;
b) determining a purification condition based on a hemagglutination assay, SDS-PAGE, or a combination thereof for influenza virus samples obtained through purification under different conditions to purify the influenza virus from the culture of step a);
c) purifying the influenza virus from the culture of step a) according to the condition determined in step b);
d) determining a detergent treatment concentration during surface antigen protein purification based on a total protein quantification assay for an influenza virus solution to purify a surface antigen from the influenza virus solution purified in step c); and
e) purifying the surface antigen protein from the influenza virus by treating the virus solution with a detergent according to the condition determined in step d).

In an aspect of the present invention, cells to be infected with a virus in step a) or cells infected with a virus in step a) may be cultured in a single-use bioreactor (SUB). A single-use bioreactor is not required to be washed and sterilized before use, thus has advantages that the process preparation time is shortened and the concern about contamination is significantly low. The single-use bioreactor may be discarded without a washing process after use, and a new single-use bioreactor may be used, thereby avoiding cross-contamination between lots or strains of the virus. In particular, the influenza vaccine is a trivalent or tetravalent vaccine, so three or four different viruses are required to be cultivated, and thus the use of a single-use bioreactor may be preferred since the process is greatly simplified and cross-contamination between strains may be prevented.

In another aspect of the present invention, before infecting cells with a virus in step a), a portion of the medium of the cell culture solution to be used for virus infection may be exchanged for a fresh medium using a low-speed continuous centrifuge. Through this, the medium may be exchanged by a method in which the cells and the medium are continuously separated from each other without a process of recovering and reintroducing the culture solution, the medium is discarded, and a new medium is introduced into the bioreactor again. Unlike a general method in which the entire amount of the medium is recovered and then divided into smaller portions to be put in containers and centrifugation is performed, when a low-speed continuous centrifuge is used, there are advantages that medium exchange can be performed in a closed system and the possibility of contamination can be greatly diminished.

In another aspect of the present invention, in the medium exchange, 50% to 90%, preferably 60% to 80%, and most preferably 70% to 80% of the old medium may be discarded, and the same amount of fresh medium may be introduced.

In another aspect of the present invention, the method for rapid purification of an influenza surface antigen may further comprise a process of treating the virus culture with a DNA-cleaving enzyme after step c). Since there is a possibility that DNA derived from the cells used for the cultivation of influenza virus is mixed in the virus culture, the process of cleaving DNA to a size of 100 bp or less by treatment with a DNA-cleaving enzyme and removing the fragments may be added. In the present invention, the type of DNA-cleaving enzyme is not particularly limited, but the DNA-cleaving enzyme may be Benzonase, Exonuclease, Ribozyme, or a mixture thereof.

In another aspect of the present invention, the method for rapid purification of an influenza surface antigen may further comprise a virus inactivation process after step c). The virus inactivation may be performed by treatment with a detergent, formaldehyde, β-propiolactone, methylene blue, psoralen, carboxyfullerene (C60), diethylamine, acetyl ethylenimine, or a combination thereof. The inactivation may be performed by physical means known to the those skilled in the art, for example, by treatment with gamma radiation and/or UV light.

The present invention also provides a method for producing an influenza virus, the method comprising:

(a) culturing a cell in a single-use bioreactor (SUB);

(b) exchanging a portion of the medium in the cell culture solution of step (a) for a fresh medium using a low-speed continuous centrifuge using a single-use bag;

(c) infecting the propagated cells with an influenza virus and culturing the cells under a condition permissive for replication of the influenza virus; and

(d) isolating the influenza virus from the culture of step (c).

In an aspect of the present invention, the cell may be Madin-Darby canine kidney (MDCK) cells.

In another aspect of the present invention, in step (b), the medium may be exchanged by a method in which the cells and the medium are continuously separated from each other using a low-speed continuous centrifuge without a process of recovering and reintroducing the entire amount of the culture solution, a portion of the medium is discarded, and a new medium is introduced into the cell incubator.

In another aspect of the present invention, the portion of the medium may be 50% to 80% of the entire culture solution.

The present invention also provides a method for manufacturing a vaccine containing an influenza antigen, the method comprising the following steps, in which a vaccine manufacture period is shortened:

(a) culturing a cell in a single-use bioreactor (SUB);

(b) exchanging a portion of the medium in the cell culture solution of step (a) for a fresh medium using a low-speed continuous centrifuge;

(c) infecting the propagated cells with an influenza virus and culturing the cells under a condition permissive for replication of the influenza virus; and

(d) isolating the influenza virus from the culture of step (c)

In an aspect of the present invention, step (d) may be performed including the following steps:

(d-1) determining a purification condition based on a hemagglutination assay for influenza virus samples obtained through purification under different conditions to purify the influenza virus from the culture of step c); and

(d-2) purifying the influenza virus from the culture of step (c) according to the condition determined in step (d-1).

In another aspect of the present invention, the method may further comprise removing cells and cell debris from the virus culture prior to step (d-1).

In another aspect of the present invention, the removal of cells and cell debris from the virus culture may be performed by one or more methods selected from the group consisting of filtration, dialysis and centrifugation.

In another aspect of the present invention, in step (d-1), the purification condition may be determined by performing the hemagglutination assay, SDS-PAGE, or a combination thereof on influenza virus culture samples purified under different conditions.

In another aspect of the present invention, the method may comprise an additional purification process by one or more methods selected from the group consisting of ultrafiltration and diafiltration after step (d-2).

In another aspect of the present invention, the method may further comprise a process of treatment with Benzonase, Exonuclease, Ribozyme, or a mixture thereof after step (d).

In another aspect of the present invention, the method may further comprise a virus inactivation process after step (d).

In another aspect of the present invention, the virus inactivation may be performed by treatment with a detergent, formaldehyde, β-propiolactone, methylene blue, psoralen, carboxyfullerene (C60), diethylamine, acetyl ethylenimine, or a combination thereof.

In another aspect of the present invention, the method may further comprise the following step:
(e) isolating a surface antigen protein from the influenza virus.

In another aspect of the present invention, in step (e), a surface antigen protein may be isolated by performing detergent treatment, then performing centrifugation, and recovering the supernatant.

In another aspect of the present invention, step (e) may be performed including the following steps:

(e-1) determining a detergent treatment concentration during surface antigen protein purification based on a total protein quantification assay for an influenza virus sample to purify a surface antigen from the influenza virus purified in step (d); and

(e-2) purifying the surface antigen protein from the influenza virus of step (d) by performing detergent treatment according to the condition determined in step (e-1).

In another aspect of the present invention, the method may further comprise a detergent removing process after step (e).

In another aspect of the present invention, in step (e-1), the detergent treatment concentration may be determined by a value obtained by determining the total protein quantification value (TPQV) per unit dose of the influenza virus isolated in step (d) and then substituting the total protein quantification value into the following Equation 1:

[Equation 1]

$$\text{detergent treatment concentration} = [\{(a*TPQV(\mu g/mL))/(b\ \mu g/mL)\}/c]*d$$

(where,

> a is 0.005% (v/v) to 0.100% (v/v),
> b is 100, 200, 300, 400, 500, 600, 700, 800, or 900, and a value closest to the TPQV is selected,
> c is a concentration of detergent stock used for treatment (% (v/v)), and
> d is a dose of the sampled influenza virus to be treated with a detergent).

In another aspect of the present invention, the method may comprise an additional chromatography performing process for removal of impurities after step e).

In another aspect of the present invention, the chromatography may be TMAE (trimethyl aminoethyl) anion-exchange chromatography.

In another aspect of the present invention, the method may comprise an additional purification process by one or more methods selected from the group consisting of ultrafiltration and diafiltration after chromatography.

[Advantageous Effects]

It is possible to rapidly and reliably confirm the conditions for obtaining (purifying) the influenza surface antigen according to the method unique to the present invention without using the single radial immunodiffusion technique, which has been used as a standard test method in the production of influenza vaccines, thus the production period of influenza surface antigen subunit vaccines is significantly shortened, and in turn, there is an expected effect that it is possible to quickly cope with a situation where the new influenza is rapidly spreading by the rapid development/manufacture of vaccine.

According to the influenza virus production method of the present invention, it is possible to provide a culture process capable of minimizing the possibility of contamination and maximizing the reduction of production period and production cost by culturing cells using a single-use bag, and exchanging the medium using a low-speed continuous centrifuge using a single-use bag and then infecting the cells with a virus in the step of infecting cells with a virus. Unlike a general method in which the entire amount of the culture solution is recovered and then divided into smaller portions to be put in containers and centrifugation is performed, when a low-speed continuous centrifuge using a single-use bag is used, medium exchange can be performed in a closed system and the possibility of contamination can be greatly diminished.

[Brief Description of Drawings]

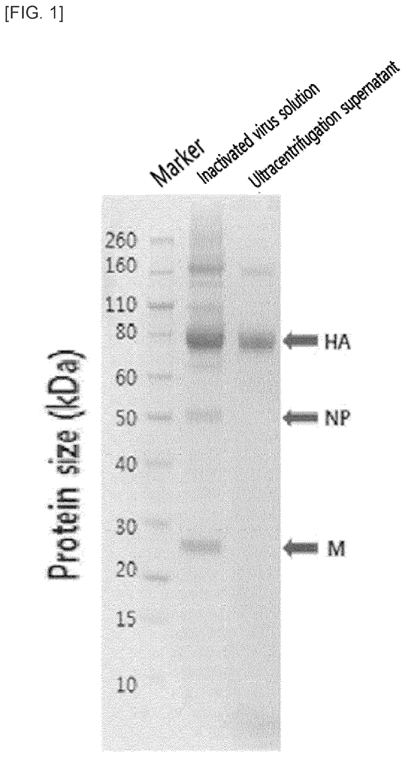

FIG. 1 is a result of SDS-PAGE analysis of a protein contained in a supernatant or inactivated virus solution obtained by determining a CTAB treatment concentration for a virus (B/Maryland/15/2016) based on a protein content according to a method of the present invention, treating an inactivated virus culture solution with CTAB, and then performing ultracentrifugation (HA: hemagglutinin, NP: nucleoprotein, M: matrix protein);

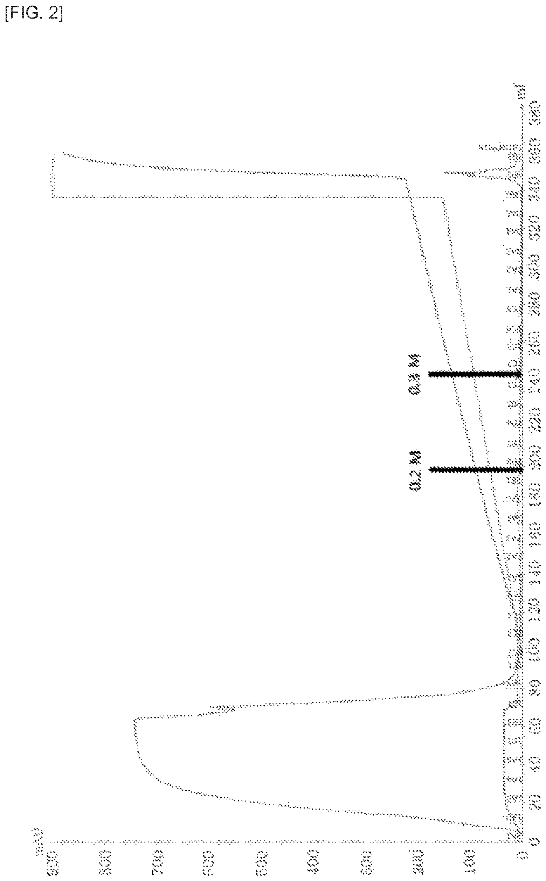

FIG. 2 is a result of confirming the conductivity condition that minimize the loss of hemagglutinin into a column flowthrough by performing linear gradient purification process and then SDS-PAGE and HA assay in order to determine an optimal condition for virus binding to CS column resin (IVR-190 virus, linear gradient purification - chromatogram);

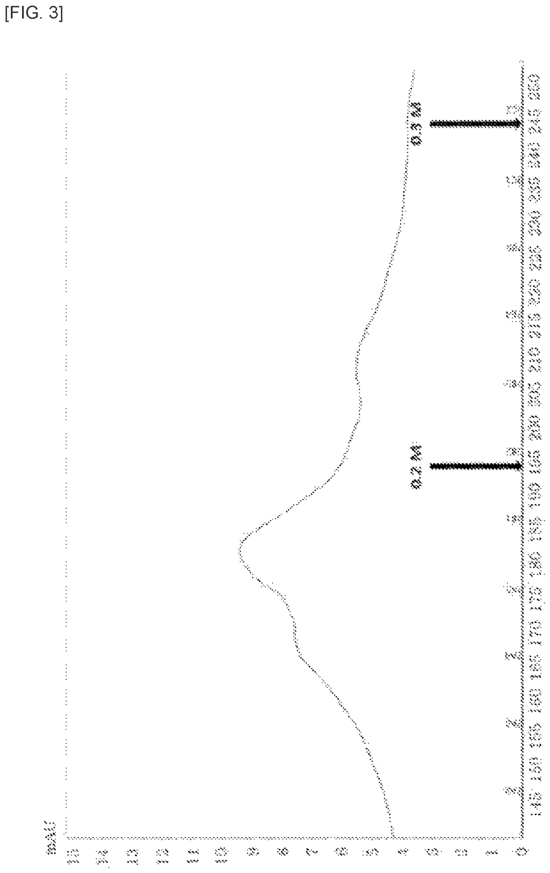

FIG. 3 is a result of confirming the conductivity condition that minimize the loss of hemagglutinin into a column flowthrough by performing linear gradient purification process and then SDS-PAGE and HA assay in order to determine an optimal condition for virus binding to CS column resin (IVR-190 virus, linear gradient purification - chromatogram (eluate fraction enlarged));

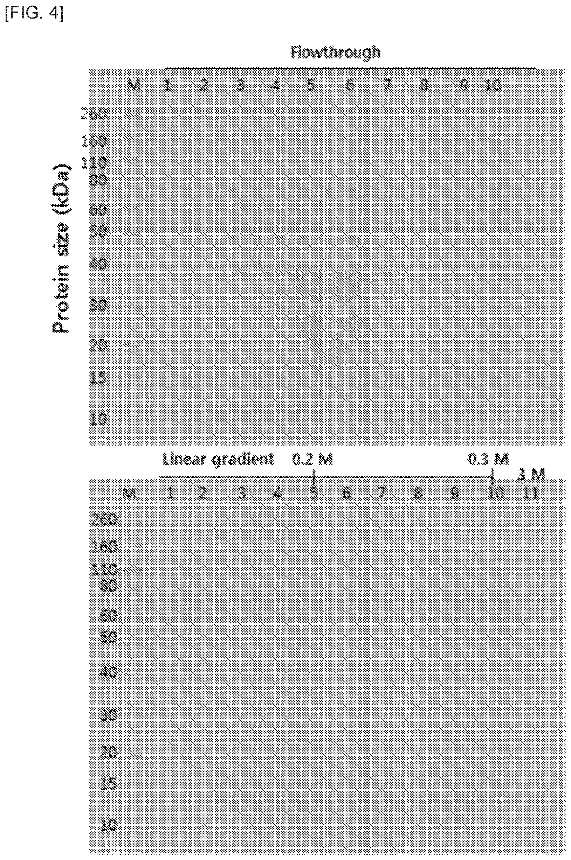

FIG. 4 is a result of confirming the conductivity condition that minimize the loss of hemagglutinin into a column flowthrough by performing linear gradient purification process and then SDS-PAGE and HA assay in order to determine an optimal condition for virus binding to CS column resin (IVR-190 virus, linear gradient purification - SDS-PAGE result); and

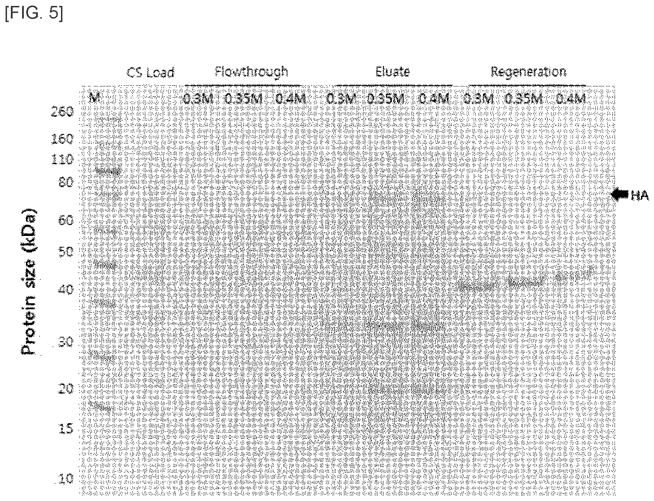

FIG. 5 is a result of analyzing the virus eluate under each condition by the HA assay and SDS-PAGE used in the method of the present invention.

**[Detailed Description of the Invention]**

Hereinafter, the present invention will be described in detail.

However, the following Examples are only illustrative of the present invention, and the contents of the present invention are not limited to the following Examples.

**Example 1: Establishment of novel strategy for influenza virus cultivation process using single-use bag and low-speed continuous centrifuge unique to the present invention**

1) Culture of MDCK cell

The culture method of the present invention provides a method for efficiently culturing MDCK Sky-3851 cells using a single-use cell incubator using a single-use bag.

An aspect of the present invention is a culture method comprising culturing MDCK cells using a spinner flask in an incubator at 37°C and 5% $CO_2$. The stirring speed in a 125 mL spinner flask is required to be set to 40 rpm to 150 rpm, and the stirring speed in 500 mL and 1 L spinner flasks is required to be set to 80 rpm to 150 rpm. As the medium used for culture, a chemically defined serum-free medium is used, and L-glutamine as a nutrient required for cell growth is added at a concentration of 3 mM to 6 mM. Culture using spinner flasks is carried out in a volume of 1 L or less.

An additional aspect of the present invention is culture through a cell incubator using a single-use bag subsequent to the flask culture, the scale of the culture solution may be increased by using a cell incubator of 10 L, 50 L, 200 L, or 2,000 L scale. Cell culture is carried out under favorable conditions and culture is performed while adjusting and maintaining culture conditions such as dissolved oxygen and pH to enable mass culture. The dissolved oxygen and pH are adjusted using the gas (air, oxygen, carbon dioxide, or nitrogen) and base (sodium bicarbonate) required for culture. In a specific aspect, the temperature, dissolved oxygen concentration, and pH during the culture step are maintained at 37°C $\pm$ 3°C, 50% $\pm$ 10% DO, and pH 7.2 $\pm$ 0.2, respectively, and the medium in the main culture contains L-glutamine at 3 mM to 6 mM.

2) Virus infection

The present invention is to obtain a virus culture solution by infecting the cell culture solution secured in the main culture step with an influenza virus. When a sufficient number of cells is secured through the main culture, the culture medium is exchanged before the cells are infected with the virus.

The medium exchange before virus infection is performed by a method in which the cells and the medium are continuously separated from each other using a low-speed continuous centrifuge using a single-use bag without a process of recovering and reintroducing the culture solution, a certain amount (70% to 80%) of the medium is discarded, and a new medium is introduced into the cell incubator again. Unlike a general method in which the entire amount of the culture solution is recovered and then divided into smaller portions to be put in containers and centrifugation is performed, when a low-speed continuous centrifuge using a single-use bag is used, medium exchange can be performed in a closed system and the possibility of contamination can be greatly diminished. Contamination generated in the process step leads to waste of the medium used in the culture step and extension of the process period, resulting in time and economic loss.

In the present invention, there is established a culture process capable of minimizing the possibility of contamination and maximizing the reduction of production period and production cost by culturing cells using a single-use bag, and exchanging the medium using a low-speed continuous centrifuge and then infecting the cells with a virus in the step infecting cells with a virus. This type of cell culture and virus infection process is referred to as a "virus cultivation process using single-use bioreactor and low-speed continuous centrifuge (abbreviation: SBCC)".

The medium exchange and virus infection step is a step of removing the medium in which the nutrients have been consumed during the main culture and introducing a new medium and a virus from the outside, is a step having the highest risk of contamination. If contamination occurs in the medium exchange step, damage to the production period occurs. It takes about 13 days from the cell thawing step of thawing the MDCK-Sky3851 cell line for vaccine production that is stored frozen to the medium exchange in the case of performing virus infection in a 10 L cell incubator. In the case of 2,000 L commercial production, it takes about 24 days to the medium exchange for virus infection. The production cost also increases because of the additional purchase of medium and single-use bags.

After medium exchange using the SBCC process, a virus strain for manufacture is prepared, the virus in the amount calculated to have a predetermined multiplicity of infection (MOI) and trypsin at a concentration of 2.5 μg/mL to 20 μg/mL are aseptically introduced into the cell incubator, the cells are infected and cultured for about 2 to 4 days, virus cultivation is terminated when the virus titer, cell number, and viability reach appropriate levels, and the culture solution is recovered. In Example of the present invention, MDCK cells having a secured cell number of $3 \times 10^6$ cells/mL or more were treated with a virus in an amount calculated to have an MOI of 0.0001 to 0.01 and trypsin at a concentration of 1 μg/mL to 20 μg/mL, and cultured for about 3 days.

**Example 2: Novel establishment of rapid search strategy for purification condition during purification process of surface antigen from influenza virus**

During the purification process of a surface antigen from an influenza virus, a detergent (ionic or non-ionic) may be added to the influenza virus, and the surface antigen protein may be isolated and purified through fractionation means such as centrifugation. At this time, as an example, it is common to use an ionic detergent such as cetyl trimethyl ammonium bromide (CTAB).

As for the detergent treatment concentration for treatment of an influenza virus, it is preferable to determine the treatment concentration depending on the content of hemagglutinin, a virus surface antigen. However, it takes about 2 to 3 days to perform the standard HA content test method SRID (single radiation immunodiffusion) generally used at present, thus SRID is not suitable as an in-process confirmation test, and has a disadvantage that the test cannot be performed particularly when the supply of SRID standard products from NIBSC and the like is not made or delayed.

Meanwhile, during the purification process of a surface antigen from an influenza virus, the detergent treatment concentration that can selectively isolate only hemagglutinin and neuraminidase, surface antigens without destroying the viral core is different for each virus. A surface antigen vaccine is a high-quality vaccine with few side effects and high purity, and it is greatly important to set an appropriate detergent treatment concentration. When an excessive amount of detergent is used for treatment, the whole virus is destroyed, and the protein inside the viral core, which is an impurity, is exposed to the outside, resulting in a decrease in purity of the surface antigen in the purified product. When the detergent treatment concentration is insufficient, most surface antigens are present in the pellet and the yield of surface antigens decreases. It is an important factor in the production of influenza vaccines to confirm the appropriate detergent treatment concentration in a short time.

The present inventors have first invented the method of the present invention to be described later after various comparative experiments utilizing various analysis techniques, and confirmed that it is possible to set the conditions for detergent treatment suitable for the rapid isolation of surface antigens without depending on SRID, as well as the method has a significant relationship with the method based on the practically existing standard test method SRID, and that reliability and reproducibility are supported. The following Examples support that the effect according to the method unique to the present invention is a special effect that is hardly predicted from the existing conventional processes. The following Examples typically show an example of using CTAB as a detergent, and this is abbreviated as CTCCT (CTAB treatment concentration confirmation test).

2-1. Novel establishment of SRID-independent surface antigen purification process condition setting method

The present inventors have derived an equation relationship as shown in Equation 1 below by implementing an algorithm for estimating the optimized detergent (here, CTAB as an example) treatment concentration during surface antigen purification using the total protein quantification value (TPQV) per unit dose of influenza virus sampled from an inactivated influenza virus concentrate. The total protein quantification value (total protein content value) was able to be measured within 1 hour by performing BCA assay (or Lowry assay, Bradford assay or the like). The reference total protein quantification value may vary depending on the strain of the virus, but the protein content in the inactivated virus concentrate used in the experiment of the present study was confirmed to be 200 **μg/mL** to 600 μg/mL through analysis. After measurement of the protein content in the inactivated virus solution, among the values of 100 **μg/mL, 200 μg/mL, 300 μg/mL, 400 μg/mL,** 500 **μg/mL, 600 μg/mL, 700 μg/mL, 800 μg/mL,** and 900 μg/mL, the value corresponding to the approximate value of the measured TPQV was set as the reference value of the protein content for CTAB treatment. The CTAB treatment concentration was determined according to Equation 1 below, and the CTAB treatment concentration (%) with respect to the protein content in the inactivated virus solution was determined within 0.005% (v/v) to 0.100% (v/v).

[Equation 1]

detergent treatment concentration = [{(a*TPQV(µg/mL))/(b µg/mL)}/c]*d

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

The CTAB treatment concentration (%) based on the protein content varies depending on the strain and virus properties, and SDS-PAGE was performed in an arbitrary range to confirm and determine the appropriate CTAB treatment concentration (%). In other words, the inactivated virus solution was divided into small portions and treated with CTAB in an arbitrary range, and then ultracentrifugation was performed at 30,000 rpm or more according to the Sky Cell Flu manufacturing process to confirm the optimal CTAB treatment concentration at which the surface antigen protein was recovered as a supernatant and the viral core protein was isolated as a precipitate.

The confirmed optimal CTAB treatment concentration was applied to the manufacturing process. SDS-PAGE was performed using the sample (B/Maryland/15/2016 virus) obtained by performing the above-described process, and the same result as in FIG. 1 was obtained. It was confirmed that impurities other than hemagglutinin found in the inactivated virus solution, such as nucleoprotein and matrix protein, when the treatment was performed with CTAB at a concentration of 0.01% to 0.10% were removed when the ultracentrifugation process was performed after the CTAB treatment, and only hemagglutinin was recovered as the ultracentrifugation supernatant.

2-2. Confirmation of reliability and reproducibility of surface antigen purification process condition setting method according to the present invention - Correlation with method based on existing SRID

[Table 1]

| Virus | Protein content (µg/mL) | CTAB treatment concentration to protein content (%) | Volume of inactivated virus solution (mL) | 5% CTAB treatment amount (mL) | HA content in inactivated virus solution (µg/mL) | HA content in supernatant after CTAB treatment | CTAB amount (g) reacted with HA (1 g) |
|---|---|---|---|---|---|---|---|
| NYMCX-275 (A/Michigan/ 45/2015 (H1N1)pdm09-like virus) | 418 | 0.08 | 160,000 | 2,675 | 75 | 50 | 11.15 |
| | 402 | 0.08 | 160,000 | 2,573 | 68 | 57 | 11.82 |
| IVR-190 (A/ Brisbane/ 02/2018 (H1N1pdm09-like virus) | 459 | 0.08 | 160,000 | 2,350 | 55 | 44 | 13.35 |
| | 458 | 0.08 | 160,000 | 2,345 | 56 | 42 | 13.09 |
| IVR-195 (A/ Kansa/14/2017 (H3N2)-like virus) | 567 | 0.06 | 200,000 | 2,268 | 103 | 77 | 550 |
| | 554 | 0.06 | 200,000 | 2,216 | 102 | 73 | 5.43 |
| B/Phuket/ 3073/2013 | 543 | 004 | 200,000 | 1,738 | 128 | 95 | 339 |
| | 542 | 004 | 200,000 | 1,734 | 134 | 100 | 324 |

(continued)

| Virus | Protein content (μg/mL) | CTAB treatment concentration to protein content (%) | Volume of inactivated virus solution (mL) | 5% CTAB treatment amount (mL) | HA content in inactivated virus solution (μg/mL) | HA content in supernatant after CTAB treatment | CTAB amount (g) reacted with HA (1 g) |
|---|---|---|---|---|---|---|---|
| B/Maryland/ 15/2016 (B/ Colorado/ 06/2017-like virus) | 450 | 004 | 200,000 | 1,800 | 158 | 114 | 2.85 |
| | 369 | 004 | 200,000 | 1,476 | 130 | 108 | 2.84 |

Table 1 is a summary of 2,000 L scale commercial production data. According to Equation 1, the detergent treatment concentration was calculated based on the protein content, and the CTAB treatment process was performed.

In addition, the HA content in the inactivated virus solution and the HA content in the ultracentrifugation supernatant obtained by performing the ultracentrifugation process after treatment of the inactivated virus solution with CTAB were obtained and compared with each other, as a result, the HA content in the ultracentrifugation supernatant was a level of 70% to 80% of the HA content in the inactivated virus solution, and sufficient commercial feasibility was confirmed. When CTAB is excessively added for the treatment in order to increase the HA recovery rate, the vaccine may be manufactured in the form of a split vaccine rather than the intended subunit vaccine, and it is thus greatly important to determine a proper CTAB treatment concentration.

Accordingly, it has been confirmed that the detergent treatment concentration calculation method according to Equation 1 established in the present invention is a method that can rapidly and accurately confirm the CTAB treatment concentration required in the purification process of surface antigens of influenza viruses.

2-3. Comparison of execution time by purification condition setting based on existing SRID (Comparative Example 1) and execution time by purification condition setting according to the present invention

As carried out in Example 2-2, the process execution time by the surface antigen purification condition setting method by the conventional SRID (single radial immunodiffusion) and the process execution time by the purification condition setting method of the present invention (see Example 2-1 and Example 2-2) were compared with each other, as a result, the execution time by the method of the present invention was much shorter than that by the conventional single radial immunodiffusion as shown in Table 2 below, and the method of the present invention was confirmed to be more efficient in confirming the influenza surface antigen purification condition.

[Table 2]

| Division | Base analytical method for condition setting | Time for standard antibody fabrication | Time for sample analysis | Total time required |
|---|---|---|---|---|
| Comparative Example 1 | SRID | 2 to 3 months | 2 to 3 days | (2 to 3 months) + (2 to 3 days) |
| Present invention | Total protein quantification assay (*e.g.*, BCA assay) | N/A | 1 hour | 3 hours |
| | SDS-PAGE | N/A | 2 hours | |

**Example 3: Establishment of rapid influenza virus antigen purification condition test set (abbreviated RIVPCT) after virus cultivation**

In the production process of a subunit vaccine containing an influenza surface antigen, the purification process requires largely two process steps of a purification process of an influenza virus from a cell culture infected with the influenza virus and an isolation process of a surface antigen protein from the influenza virus. In the existing vaccine production process, the process conditions may be optimized using the standard test method single radial immunodiffusion (SRID) in the two process steps, but SRID has several disadvantages in vaccine production as described above. Accordingly, the present inventors have searched for a novel method that can set suitable process conditions without single radial immunodiffusion (SRID), and developed a rapid influenza virus antigen purification condition test set including the following first purification condition determination method and second purification condition determination method (based on the new strategy established in Example 2) as a set. In the present invention, the set of the first purification condition determination method and the second purification condition determination method is herein referred to as RIVPCT.

3-1. First purification condition determination method - Case of isolation/purification/concentration of influenza virus from influenza virus culture

There is provided a determination method/discrimination method for rapidly setting optimal conditions for each virus without SRID measurement in setting the purification conditions used in the step of purifying the influenza virus from an influenza virus culture. Specifically, there is provided a test method/judgment method for determining the conditions for purification of the influenza virus from the influenza virus samples obtained through purification under different conditions based on the hemagglutination assay, SDS-PAGE, and discrimination criteria unique to the present invention, which will be described later.

In the following Examples, an example using chromatography is shown as a representative virus purification means. Before the virus culture solution is introduced into the chromatography column, a pretreatment in which cells and cell debris are primarily removed from the virus culture solution may be performed, such pretreatment may be performed by, for example, centrifugation, dialysis, filtration, or the like.

In order to perform the chromatography process, it is necessary to set different conditions for each influenza virus, and it is necessary to rapidly confirm the purification conditions for each virus strain because of the nature of influenza viruses, where the recommended strain for vaccine production is changed every year. In order to set the chromatography purification condition, the condition under which the influenza virus can bind to the resin most favorably is confirmed, and then a test for the elution condition under which the bound virus can be isolated and purified with optimal yield and purity is performed. Hereinafter, a method for determining influenza virus purification conditions (binding conditions and elution conditions of the target substance to the CS chromatography column resin) in the case of using Cellufine Sulfate (CS) chromatography is described as a representative example, which is abbreviated as CSPCT (CS chromatography purification condition test).

The principle of virus binding to the resin of the chromatography column is ionic bonding due to the affinity of the surface protein of influenza virus for the sulfate group of resin, and the binding capacity is generally determined depending on conductivity and pH. In the present Example, it is exemplarily shown that conditions under which the maximum number of viruses can bind to the resin are searched while maintaining the pH of the buffer used in the process at a neutral pH of 7.0 to 7.4 to increase the protein stability and lowering the conductivity. The conductivity of a generally obtained influenza virus culture (solution) is about 10.0 mS/cm to 15.0 mS/cm, and the binding capacity is confirmed while lowering the conductivity of the sample to be loaded on the column by diluting the influenza virus culture (solution) with a sodium phosphate buffer having a low conductivity. In the present Example, it is exemplarily shown that the time and variables required for confirming the purification conditions are diminished in a simple manner of adjusting only the conductivity, except for the pH, which is generally set as one of the purification conditions in order to rapidly confirm the process conditions.

In general, it is known that the influenza virus binds to the CS resin more favorably as the conductivity is lower, but the volume of the sodium phosphate buffer to be introduced to lower the conductivity and the time for the CS chromatography process increase, this increases the production period and production cost, and it is thus important to confirm the proper conductivity. Therefore, while lowering the conductivity to 10.0 mS/cm or less in a manner of diluting the virus culture solution 0.5 to 4 times using a sodium phosphate buffer, the hemagglutination assay (HA assay) was performed on the column flowthrough to confirm whether the binding capacity was proper.

Specifically, the HA assay was performed according to the protocol described in the "Manual for the laboratory diagnosis and virological surveillance of influenza" prepared and distributed by the WHO. According to the method, the proper conductivity of the loading sample was confirmed based on the trend of decreasing the HA titer of the column flowthrough, and SDS-PAGE was also performed at the same time to confirm whether the influenza virus was lost into the column flowthrough for each condition.

After the optimal conditions for virus binding to the CS column resin have been determined as described above, conditions suitable for virus elution are searched. The elution of the influenza virus bound to the resin was performed in a manner of searching for the optimal conductivity using a buffer solution containing sodium chloride at an appropriate concentration to cleave binding to the resin. When virus culture solution is introduced into the column while increasing the salt concentration with 2 M to 5 M sodium chloride buffer under a linear gradient condition, a UV peak appears as the viruses, proteins and the like are eluted. These fractions were recovered and the conductivity and salt concentration in the fraction containing the virus were confirmed by performing HA assay and SDS-PAGE. Based on the virus elution conditions confirmed here and the conductivity conditions under which the virus binding capacity was proper confirmed above, the virus was eluted under a step gradient condition using sodium chloride buffer and sodium phosphate buffer to confirm whether most influenza viruses were eluted at the corresponding elution concentration, and then virus elution conditions were established. In order to confirm whether the virus is eluted with high yield and high purity, the HA assay was performed by the same method and criteria as those described above. SDS-PAGE was also performed for the confirmation.

The CS chromatography purification condition test (CSPCT) for purifying the virus from the recovered virus culture solution, which is performed in a series of processes as described above, can be completed within about 6 to 12 hours, and such time reduction is also related to the reduction of the overall production period because of the nature of influenza vaccine, where the strain for vaccine production is changed every year.

When the chromatography conditions are set, the most accurate method is to confirm the hemagglutinin content in the column flowthrough by performing the standard test method, single radial immunodiffusion technique (hereinafter, SRID), but it takes 2 to 3 days to confirm one purification condition, greatly increasing the condition test period, and the SRID technique has a disadvantage that the test cannot be performed when the supply of SRID standard products from NIBSC (National Institute for Biological Standards and Control), TGA (Therapeutic Goods Administration) and the like is not made or delayed. When the HA assay and SDS-PAGE are performed according to the present invention, the test result can be acquired within 3 hours, thus the purification condition test for each virus is rapidly done within 1 day, and there are few restrictions on test execution since there are no issues such as the supply of standard products. It can be said that the characteristics of above-described CSPCT are the method in which the binding capacity of an influenza virus is confirmed by appropriately lowering the conductivity and the conditions for eluting the virus with high yield and high purity are rapidly confirmed within 12 hours, and the principles and procedures thereof are the same as those described above.

3-2. Second purification condition determination method - Isolation and purification of surface antigen from influenza virus

For the isolation and purification of surface antigens from an influenza virus, an example of using CTAB as a detergent is representatively presented in the present Example. CTAB treatment concentration was rapidly confirmed by performing CTCCT in RIVPCT in the same manner as in Example 2.

3-3. Purification effect of surface antigen protein according to first purification condition determination method and second purification condition determination method

In the case of determining the necessary conditions for the purification processes required for vaccine production based on the HA content confirmed by performing SRID, more accurate purification conditions can be set. However, according to the RIVPCT of the present invention, there is an advantage that conditions necessary for the purification processes required for vaccine production can be identified more rapidly. When performing the method using SRID, it takes 2 days or longer even if the possession of standard products and the period of stock are not taken into consideration. In order to shorten the time required, the RIVPCT method using SDS-PAGE, HA assay and the like has been invented.

[Table 3]

| | Setting of condition for purifying virus from virus culture solution (CSPCT) | | Setting of condition for isolating surface antigen from virus (CTCCT) | |
|---|---|---|---|---|
| | Base analytical method for condition setting | Total time required for condition analysis | Base analytical method for condition setting | Total time required for condition analysis |
| Setting method using SRID | SRID | (2 to 3 months) + (2 to 3 days) | SRID | (2 to 3 months) + (2 to 3 days) |
| Present invention (RIVPCT) | HA assay, SDS-PAGE | 6 to 12 hours | Total protein quantification assay (*e.g.*, BCA assay), SDS-PAGE | 3 hours |

**Example 4: Influenza subunit vaccine production process with reduced production period**

4-1. Infection of cell with influenza virus and culture of cell

1) Culture of MDCK (Madin-Darby canine kidney) cells

One vial of the MDCK-Sky3851 cell line for vaccine production kept frozen was thawed in a 37°C constant temperature water bath and diluted with a culture medium. Next, the diluted cells were centrifuged to separate the cells from the medium, and then the cell pellet was again released with a fresh medium, a sample was taken, and the cell number and viability were measured. The cells were inoculated in a 125 mL spinner flask at an inoculation concentration of $1\times10^5$ cells/mL to $5\times10^5$ cells/mL, and cultured for 3 to 4 days at 37°C, 5% $CO_2$ with stirring at 80 rpm.
Then cell number and viability of MDCK cells being cultured in the 125 mL spinner flask were measured, the cell culture solution was inoculated sufficiently to start culture on a 250 mL scale in a 500 mL spinner flask, and the primary seed culture was performed for 3 to 4 days at 37°C and 5% $CO_2$ with stirring at 100 rpm. The cells were cultured in a batch process, a chemically defined medium was used as a cell culture medium, and L-glutamine was added at a concentration of 3 mM/L to 6 mM/L. After the primary seed culture, the cell number and viability were measured, and then cells were inoculated sufficiently to start culture on a 500 mL scale in two 1 L spinner flasks, and cultured for 3 to 4 days under the same conditions as in the primary seed culture.
A sample was taken from the 1 L spinner flask in culture on a 500 mL scale, and the number of cells and viability were measured, and then the cells of the culture solution in the 1 L spinner flask were aseptically inoculated into the cell incubator sufficiently to start culture on a 5 L scale in a 10 L single-use cell incubator, and cultured for 3 to 4 days. The necessary instruments and pH and DO probes were prepared and sterilized in advance to inoculate the cell culture solution in the spinner flask into the cell incubator. The pH and DO probes were installed, the culture medium was introduced, then the temperature was set to 37°C ± 1°C, and the DO value was corrected for inoculation of cells.
In the same manner, cell culture was sequentially performed up to a 50 L scale, 100 L scale, 500 L scale, and 2,000 L scale.

2) Virus infection

When the number of cells of $1\times10^6$ cells/mL to $3\times10^6$ cells/mL or more was secured by taking the culture solution in the cell incubator being cultured on the 2,000 L scale and measuring the number of cells and the viability, the existing culture medium was replaced with a fresh medium. The culture medium was exchanged by removing 70% to 80% of the medium based on the cell culture solution using a low-speed continuous centrifuge and introducing a fresh medium again into the single-use bag for cell culture.
During the medium exchange and virus infection processes, the inside of the cell incubator is maintained in a closed environment, the material used for medium exchange is a single-use bag in a sterilized state, and thus the possibility of contamination is significantly low. The process time required for the corresponding process was 3 to 4 hours when the process was performed on a 10 L scale, and 7 to 12 hours for commercial production on a 2,000 L scale.
Virus infection and propagation was carried out under the conditions of DO 50% ± 10%, pH 7.2 ± 0.2, the virus strain for manufacture of the influenza virus to be manufactured was thawed, the virus in an amount calculated to have an MOI of 0.0001 to 0.002 and trypsin at a concentration of 2.5 **µg/mL** to 20 µg/mL were aseptically introduced into the cell incubator, the cells were infected and cultured for about 3 to 4 days, the culture was terminated when the virus titer, number of cells, and viability reached proper levels, and the culture solution was recovered.
Microcarriers were not used in the cell culture and virus infection method. In particular, cells were cultured and viruses were propagated by a batch cell culture system rather than a perfusion system. After the MDCK-Sky3851 was infected with the influenza virus, the influenza virus was propagated in the cell, burst the cell, and was ejected to the outside, and thus the perfusion system was not suitable for the system of the present invention since the number of cells gradually decreased when cells were cultured by the perfusion system.

4-2. Setting of condition for purification of influenza virus from virus culture and virus purification

1) Centrifugation and filtration

When the virus cultivation in the cell incubator was completed, cells and cell debris were removed by centrifuging the virus culture solution at a speed of 8,000 rpm to 20,000 rpm using a low-speed continuous centrifuge and recovering the supernatant. The recovered supernatant was filtered through a 1.0/0.5 µm pre-filter and then filtered again through a 0.45 µm filter to remove large particles of a certain size or more.

2) Purification condition setting and influenza virus purification using Cellufine Sulfate (CS) affinity resin

The filtered virus culture solution was subjected to a primary chromatography process using a resin (Cellufine Sulfate) having affinity for influenza virus. After the column was equilibrated using a sodium phosphate buffer for equilibration, the virus filtrate was introduced. Purification conditions were rapidly set by the CSPCT method as described in Example 3-1 above. Purification condition setting test was performed using IVR-190 (A/Brisbane/02/2018(H1N1) pdm09-like virus), and the result by the CSPCT method and the result by the method using SRID were compared with each other and analyzed.

First, in order to determine the optimal conditions for virus binding to the CS column resin, the linear gradient purification process was performed and SDS-PAGE and HA assay were performed to confirm the conductivity conditions under which the loss of hemagglutinin into the column flowthrough was minimized (FIGS. 2 to 4, Table 4).

[Table 4]

| IVR-190 virus, Linear gradient purification - HA assay result | | | | | |
|---|---|---|---|---|---|
| Flowthrough fraction | | | Peak fraction | | |
| No. | Description | HA titer (HAU/50 μL) | No. | Description | HA titer (HAU/50 μL) |
| 1 | Flowthrough fraction 1 | 0 | 1 | 0.1 M eluate-1 | 0 |
| 2 | Flowthrough fraction 2 | 0 | 2 | 0.1 M eluate-2 | 8 |
| 3 | Flowthrough fraction 3 | 0 | 3 | 0.1 M eluate-3 | 64 |
| 4 | Flowthrough fraction 4 | 0 | 4 | 0.1 M eluate-4 | 256 |
| 5 | Flowthrough fraction 5 | 0 | 5 | 0.2 M eluate | 128 |
| 6 | Flowthrough fraction 6 | 0 | 6 | 0.2 M eluate-1 | 32 |
| 7 | Flowthrough fraction 7 | 0 | 7 | 0.2 M eluate-2 | 32 |
| 8 | Flowthrough fraction 8 | 0 | 8 | 0.2 M eluate-3 | 16 |
| 9 | Flowthrough fraction 9 | 0 | 9 | 0.2 M eluate-4 | 16 |
| 10 | Flowthrough fraction pooling | 0 | 10 | 0.3 M eluate | 16 |
| | | | 11 | Regeneration | 256 |

The conductivity was lowered from 13.03 mS/cm to 7.54 mS/cm by diluting the IVR-190 virus culture solution with a sodium phosphate buffer for equilibration at 1:1, and a linear gradient purification condition test was performed. It was confirmed that on SDS-PAGE, there was no HA lost into the column flowthrough, and impurities except HA were eluted. The result of HA assay using 0.5% chicken RBC was also confirmed to be 0 HAU/50 μL in all column flow fractions, and thus it was confirmed that the conductivity of the loading sample used for the test was suitable for the primary chromatography purification.

Regarding the confirmation of the virus elution concentration, a sodium phosphate buffer for elution containing sodium chloride and a sodium phosphate buffer were mixed at an appropriate ratio and introduced, and the eluted virus fraction was collected while confirming the UV280 peak, as a result, the highest peak (UV280 value) and HA titer on the chromatogram were confirmed at the 0.1 M to 0.2 M NaCl concentration as well as the peak and HA titer were confirmed to gradually decrease until the 0.3 M NaCl concentration condition, and thus it was confirmed that 0.4 M NaCl was suitable as the virus elution concentration in order to increase the yield. Considering the test results, the appropriate conductivity of the sample to be loaded on the CS column was judged to be a level of 7.54 mS/cm, and 0. M NaCl was judged to be suitable as the virus elution concentration.

In order to compare the CSPCT method with the method based on SRID, after the conductivity condition of the loading sample was set according to the CSPCT method described above, the step gradient purification process was performed by setting the virus elution concentration to 0.3 M, 0.35 M, and 0.4 M NaCl, and then the HA contents in the virus eluates of the respective batches were compared with each other. In addition, by performing SDS-PAGE and HA assay, the correlation between the results acquired by the CSPCT method of the present invention and the results acquired by the method using SRID was confirmed.

As a result of performing SRID, it was confirmed that the yield and purity were the most favorable when the virus was eluted at a 0.4 M NaCl concentration (Table 5).

[Table 5]

| IVR-19 virus, Step gradient purification -SRID result (virus eluate) | | | |
|---|---|---|---|
| Virus elution concentration | 0.3 M NaCl | 0.35 M NaCl | 0.4 M NaCl |
| HA content in virus eluate | 3 μg/mL | 8 μg/mL | 10 μg/mL |
| Total protein content | 129 μg/mL | 130 μg/mL | 142 μg/mL |
| Purity (HA/protein) | 2.3% | 6.2% | 70% |

The HA assay and SDS-PAGE used in the CSPCT method were performed using the sample used for SRID to analyze the virus eluate under each condition.

Referring to the SDS-PAGE result of FIG. 5, it was confirmed that the HA band became thicker as the virus elution concentration increased, and through this, it was found that the HA contained in the virus eluate was increased. The HA titer of the virus eluate was the same as 1,024 HAU/50 μL under the 0.3 M and 0.35 M NaCl concentration conditions, but increased to 2,048 HAU/50 μL under the 0.4 M NaCl concentration condition. As such, in the virus elution condition confirmed by the CSPCT method as well, it was confirmed that the virus elution concentration was 0.4 M NaCl as in the method using SRID, and it was possible to confirm the correlation between the two methods (Table 6).

[Table 6]

| HA titer of virus eluate at each virus elution concentration | | | | |
|---|---|---|---|---|
| Virus elution conc ncentration | | 0.3 M NaCl | 0.35 M NaCl | 0.4 M NaCl |
| HA titer (HAU/50 μL) | Sample loaded on column | 512 | 512 | 512 |
| | Column flowthrough | 0 | 0 | 0 |
| | Virus eluate | 1,024 | 1,024 | 2,048 |
| | Regeneration | 1,024 | 1,024 | 512 |

Meanwhile, the pH of the equilibration buffer used in the purification process was maintained at a neutral pH of 7.0 to 7.4, and the virus filtrate was diluted with an equilibration buffer to lower the conductivity to the value at which the maximum number of viruses could bind. At the termination of virus cultivation, the conductivity of the virus culture solution was about 10.0 mS/cm to 15.0 mS/cm, the binding capacity of the virus to the resin was confirmed while lowering the conductivity by diluting the virus culture solution with an equilibration buffer having a low conductivity, as a result, the virus exhibited an appropriate level of binding capacity in the condition of about 4.0 mS/cm to 10.0 mS/cm although there is a difference depending on the type of influenza virus.

After the virus filtrate diluted with the equilibration buffer was introduced into the CS column, the column flowthrough that did not bind to the column but flowed out was sampled and discarded, the column was sufficiently washed by introducing the equilibration buffer by 2 to 3 CV, then a sodium phosphate buffer for elution containing sodium chloride and a sodium phosphate buffer were diluted at an appropriate ratio and introduced, and the eluted virus fraction was collected while confirming the UV280 peak. The conditions for elution of influenza virus were established by confirming the elution buffer containing sodium chloride at an appropriate concentration at which binding between virus and resin could be cleaved by performing CSPCT (refer to Example 3-1) in RIVPCT, and most viruses were eluted at a sodium chloride concentration of 1 M or less.

4-3. Concentration of virus and desalting

Primary ultrafiltration is performed to concentrate the virus eluate and exchange the virus eluate for PBS buffer. The virus eluate was concentrated to 10% or less based on the volume of cell culture solution using an ultrafiltration filter having a size of 300 kDa to 800 kDa, and diafiltration was performed with PBS buffer in a volume equal to or more than 8 times the concentrated volume until the conductivity was the same as that of the PBS buffer.

4-4. Removal of MDCK cell-derived DNA in virus concentrate

In order to remove cell line-derived DNA from the recovered virus concentrate, the recovered virus concentrate was treated with Benzonase (Merck, Inc.), a DNA cleaving enzyme at 0.5 U/mL to 50 U/mL and the reaction was conducted with slow stirring at room temperature for 30 minutes to 24 hours. To increase the activity of Benzonase, 0.5 mM to 5 mM magnesium chloride hexahydrate was added as a cofactor of Benzonase.

4-5. Virus inactivation

Infectivity was eliminated by inactivating the influenza virus concentrate subjected to the Benzonase reaction. As the chemical means for inactivating viruses, any known means can be used, and examples thereof include a detergent, formaldehyde, *beta*-propiolactone, methylene blue, psoralen, carboxyfullerene (C60), and the like. In the present Example, the virus was inactivated by treatment with a formaldehyde solution at a level of 0.01% to 0.1%.

4-6. Isolation of surface antigen using detergent

Surface antigen proteins can be isolated and purified by treating an influenza virus with a detergent (ionic or nonionic) or solvent, and as a representative example, it is common to use an ionic detergent such as cetyl trimethyl ammonium bromide (CTAB).
In the present Example as well, the virus was treated with CTAB based on the protein content in the inactivated virus concentrate, the CTAB treatment concentration was confirmed by performing CTCCT in RIVPCT as in Example 3-2. After the reaction is conducted in refrigeration or at room temperature for 1 hour or longer, centrifugation is performed at 30,000 rpm or higher using a continuous ultracentrifuge, then the supernatant is recovered, and only the surface antigen protein is isolated.
In order to remove CTAB in the ultracentrifugation supernatant, resin for adsorption (Amberlite XAD-4) was added to the supernatant to remove CTAB. Next, the resin for adsorption was removed by filtration through a filter.

4-7. Residual DNA removal using TMAE anion exchange resin

In order to further remove host cell-derived DNA, a secondary chromatography process was performed using an anion exchange chromatography resin. After the column was equilibrated using a sodium phosphate buffer for equilibration, the virus surface antigen recovery filtrate is introduced. At this time, the filtrate is diluted with a sodium phosphate buffer containing 1 M to 6 M sodium chloride and having a high conductivity, the chromatography process is performed at a conductivity at which the surface antigen does not bind to resin but DNA binds to resin, and the column flowthrough not bound to the resin is recovered as a surface antigen fraction.

4-8. Concentration of surface antigen fraction and desalting

The surface antigen fraction was concentrated using an ultrafiltration filter having a size of 30 kDa to 50 kDa. When the concentration process was completed, buffer exchange was performed using PBS buffer until the conductivity of the concentrate was the same as that of the PBS buffer, and the concentrate was recovered. The recovered concentrate was sterilized and filtered through a 0.5/0.2 μm filter to produce a stock solution, and the stock solution was stored at 2°C to 8°C.

**Industrial Applicability**

It is possible to rapidly and reliably confirm the conditions for obtaining (purifying) the influenza surface antigen according to the method unique to the present invention without using the single radial immunodiffusion technique, which has been used as a standard test method in the production of influenza vaccines, thus the production period of influenza surface antigen subunit vaccines is significantly shortened, in turn, it is possible to quickly cope with a situation where the new influenza is rapidly spreading by the rapid development/manufacture of vaccine, and the industrial applicability of the method is significantly high.

**Claims**

1. A method for purifying a surface antigen protein from an influenza virus using a detergent, wherein a detergent treatment concentration for treatment of a sampled influenza virus is determined by a value obtained by determining a total protein quantification value (TPQV) per unit dose of the sampled influenza virus and then substituting the total protein quantification value into Equation 1 as follows:

[Equation 1]

detergent treatment concentration = [{(a*TPQV(μg/mL))/(b μg/mL)}/c]*d

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

2. The method of claim 1, wherein the detergent treatment concentration is a concentration for selectively isolating only a surface antigen in a state in which a viral core is not destroyed or a state in which destruction of a viral core is minimized.

3. The method of claim 1, wherein a quantification process by SRID (single radial immunodiffusion) for an amount of hemagglutinin (HA) protein in a sample is not required in the determination of detergent treatment concentration.

4. The method of claim 1, wherein the detergent is a cationic detergent.

5. The method of claim 4, wherein the cationic detergent is CTAB (cetyl trimethyl ammonium bromide).

6. The method of claim 1, wherein the total protein quantification value (TPQV) is obtained by BCA assay, Lowry assay, or Bradford assay.

7. The method of claim 1, wherein the sampled influenza virus is obtained by a method including the following steps:

(a) culturing a cell in a single-use bioreactor (SUB);
(b) exchanging a portion of the medium in the cell culture solution of step (a) for a fresh medium using a low-speed continuous centrifuge using a single-use bag;
(c) infecting the propagated cells with an influenza virus and culturing the cells under a condition permissive for replication of the influenza virus; and
(d) isolating the influenza virus from the culture of step (c).

8. The method of claim 7, wherein the cell is Madin-Darby canine kidney (MDCK) cells.

9. The method of claim 7, wherein the medium is exchanged by a method in which the cells and the medium are continuously separated from each other using a low-speed continuous centrifuge, a portion of the medium is discarded, and a new medium is introduced into the cell incubator in step (b), without a process of recovering and reintroducing an entire amount of the culture solution.

10. The method of claim 9, wherein a portion of the medium is 50% to 80% of the entire culture solution.

11. The method of claim 7, wherein step (d) is performed including the following steps:

(d-1) determining a purification condition based on a hemagglutination assay for influenza virus samples obtained through purification under different conditions to purify the influenza virus from the culture of step c); and
(d-2) purifying the influenza virus from the culture of step (c) according to the condition determined in step (d-1).

12. The method of claim 1, wherein the total protein quantification value per unit dose of the sampled influenza virus is 50 **μg/mL** to 950 μg/mL.

13. A test method for rapid confirmation of influenza antigen purification conditions, the method comprising the following first purification condition determination method and second purification condition determination method:

the first purification condition determination method, which is used in a step of purifying an influenza virus from an influenza virus culture, and in which a condition for the purification of an influenza virus is determined based on a hemagglutination assay, SDS-PAGE, or a combination thereof for influenza virus samples obtained through purification under different conditions; and

the second purification condition determination method, which is used in a step of purifying a surface antigen protein from an influenza virus using a detergent, and in which a detergent treatment concentration during the purification of a surface antigen protein is determined based on a total protein quantification assay for an influenza virus sample.

14. The method of claim 13, wherein the test method for rapid confirmation of influenza antigen purification conditions does not require a quantification process by SRID (single radial immunodiffusion) for an amount of hemagglutinin (HA) protein in an influenza virus.

15. The method of claim 13, wherein the step of purifying an influenza virus from the influenza virus culture is performed by chromatography.

16. The method of claim 15, wherein the chromatography is performed using a chromatograph packed with Cellufine Sulfate (CS).

17. The method of claim 13, wherein the conditions include buffer type, buffer concentration, pH, and conductivity.

18. The method of claim 13, wherein the conditions are conductivity.

19. The method of claim 13, wherein in the second purification condition determination method is **characterized in that** the detergent treatment concentration is determined by a value obtained by determining a total protein quantification value (TPQV) per unit dose of the influenza virus culture obtained through the first purification and then substituting the total protein quantification value into Equation 1 as follows:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the influenza virus culture to be treated with a detergent).

20. A vaccine manufacturing method comprising purifying a surface antigen protein from an influenza virus, wherein the step is carried out under a condition of using a detergent for treatment in a value obtained by determining a total protein quantification value (TPQV) per unit dose of the sampled influenza virus and then substituting the total protein quantification value into Equation 1 as follows:

[Equation 1]

$$\text{detergent treatment concentration} = [\{(a*TPQV(\mu g/mL))/(b\ \mu g/mL)\}/c]*d$$

(where,

a is 0.005% (v/v) to 0.100% (v/v),
b is 100, 200, 300, 400, 500, 600, 700, 800, or 900, and a value closest to the TPQV is selected,
c is a concentration of detergent stock used for treatment (% (v/v)), and
d is a dose of the sampled influenza virus to be treated with a detergent).

21. A method for rapid purification of an influenza surface antigen, the method comprising the following steps:

a) infecting a cell with an influenza virus and culturing the cell to obtain a virus culture;

b) determining a purification condition based on a hemagglutination assay, SDS-PAGE, or a combination thereof for influenza virus samples obtained through purification under different conditions to purify the influenza virus from the culture of step a);

c) purifying the influenza virus from the culture of step a) according to the condition determined in step b);

d) determining a detergent treatment concentration during surface antigen protein purification based on a total protein quantification assay for an influenza virus to purify a surface antigen from the influenza virus purified in step c); and

e) purifying the surface antigen protein from the influenza virus by performing detergent treatment according to the condition determined in step d).

22. The method of claim 21, wherein the method for rapid purification of an influenza surface antigen further comprises removing cells and cell debris from the virus culture prior to step b).

23. The method of claim 22, wherein the removal of cells and cell debris from the virus culture is performed by one or more methods selected from the group consisting of filtration, dialysis and centrifugation.

24. The method of claim 21, wherein the method for rapid purification of an influenza surface antigen comprises an additional purification process by one or more methods selected from the group consisting of ultrafiltration and diafiltration after step c).

25. The method of claim 21, wherein the surface antigen protein is isolated by performing detergent treatment, then performing centrifugation, and recovering a supernatant in step e).

26. The method of claim 21, wherein the method for rapid purification of an influenza surface antigen further comprises a detergent removing process after step e).

27. The method of claim 21, wherein the method for rapid purification of an influenza surface antigen comprises an additional chromatography performing process for removal of impurities after step e).

28. The method of claim 27, wherein the chromatography is TMAE (trimethyl aminoethyl) anion exchange chromatography.

29. The method of claim 28, wherein the method for rapid purification of an influenza surface antigen comprises an additional purification process by one or more methods selected from the group consisting of ultrafiltration and diafiltration after chromatography.

30. The method of claim 21, wherein a cell to be used for virus infection in step a) or a cell infected with a virus in step a) is cultured in a single-use bioreactor (SUB).

31. The method of claim 21, wherein a portion of the medium in the cell culture to be used for virus infection is exchanged for a fresh medium through a low-speed continuous centrifuge before a cell is infected with a virus in step a).

32. The method of claim 21, wherein the method for rapid purification of an influenza surface antigen further comprises a process of treatment with Benzonase, Exonuclease, Ribozyme, or a mixture thereof after step c).

33. The method of claim 21, wherein the method for rapid purification of an influenza surface antigen further comprises a virus inactivation process after step c).

34. The method of claim 33, wherein the virus inactivation is performed by treatment with a detergent, formaldehyde, $\beta$-propiolactone, methylene blue, psoralen, carboxyfullerene (C60), diethylamine, acetyl ethylenimine, or a combination thereof.

[FIG. 1]

[FIG. 2]

[FIG. 3]

[FIG. 4]

Flowthrough

[FIG. 5]

## INTERNATIONAL SEARCH REPORT

| International application No. |
| --- |
| **PCT/KR2021/004356** |

| A. | CLASSIFICATION OF SUBJECT MATTER |
| --- | --- |

**C07K 14/005**(2006.01)i; **C12M 1/00**(2006.01)i; **C12N 7/00**(2006.01)i

According to International Patent Classification (IPC) or to both national classification and IPC

| B. | FIELDS SEARCHED |
| --- | --- |

Minimum documentation searched (classification system followed by classification symbols)

C07K 14/005(2006.01); A61K 39/145(2006.01); C07K 1/14(2006.01); C07K 1/20(2006.01); C07K 1/34(2006.01); C07K 1/36(2006.01); C07K 14/11(2006.01); G01N 33/569(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인플루엔자 바이러스(influenza virus), 정제 조건(purification condition), 계면활성제(detergent), 적혈구 응집 반응법(hemagglutination assay), SDS-PAGE, 항원(antigen)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | KR 10-2012-0030442 A (NOVARTIS AG) 28 March 2012 (2012-03-28)<br>See abstract; claims 1 and 16; and paragraph [0050]. | 1-34 |
| A | KR 10-2016-0096215 A (ABBOTT BIOLOGICALS B.V.) 12 August 2016 (2016-08-12)<br>See entire document. | 1-34 |
| A | US 2007-0196387 A1 (KAPTEYN, J. C. et al.) 23 August 2007 (2007-08-23)<br>See entire document. | 1-34 |
| A | KR 10-2015-0060767 A (DENKA SEIKEN CO., LTD.) 03 June 2015 (2015-06-03)<br>See entire document. | 1-34 |
| A | LEE, K. K. H. et al. Quantitative determination of the surfactant-induced split ratio of influenza virus by fluorescence spectroscopy. Human Vaccines & Immunotherapeutics. 2016, vol. 12, no. 7, pp. 1757-1765.<br>See entire document. | 1-34 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 July 2021** | **07 July 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## EP 4 144 753 A1

### INTERNATIONAL SEARCH REPORT
#### Information on patent family members

International application No.

**PCT/KR2021/004356**

| Patent document cited in search report | | | Publication date (day/month/year) | Patent family member(s) | | | Publication date (day/month/year) |
|---|---|---|---|---|---|---|---|
| KR | 10-2012-0030442 | A | 28 March 2012 | AU | 2010-252661 | A1 | 22 December 2011 |
| | | | | AU | 2010-252661 | B2 | 22 August 2013 |
| | | | | CA | 2763440 | A1 | 02 December 2010 |
| | | | | CN | 103764160 | A | 30 April 2014 |
| | | | | EA | 201171491 | A1 | 30 July 2012 |
| | | | | EP | 2435066 | A1 | 04 April 2012 |
| | | | | EP | 2435066 | B1 | 20 November 2013 |
| | | | | HK | 1168795 | A1 | 11 January 2013 |
| | | | | JP | 2012-528139 | A | 12 November 2012 |
| | | | | JP | 2015-098479 | A | 28 May 2015 |
| | | | | JP | 5823379 | B2 | 25 November 2015 |
| | | | | NZ | 596671 | A | 28 June 2013 |
| | | | | US | 2012-0237545 | A1 | 20 September 2012 |
| | | | | WO | 2010-136896 | A1 | 02 December 2010 |
| KR | 10-2016-0096215 | A | 12 August 2016 | AU | 2012-286098 | A1 | 23 January 2014 |
| | | | | AU | 2012-286098 | B2 | 12 May 2016 |
| | | | | AU | 2012-286098 | C1 | 15 September 2016 |
| | | | | BR | 112014001049 | A2 | 21 February 2017 |
| | | | | CA | 2841604 | A1 | 24 January 2013 |
| | | | | CA | 2841604 | C | 21 January 2020 |
| | | | | CN | 103827296 | A | 28 May 2014 |
| | | | | CN | 103827296 | B | 16 November 2016 |
| | | | | EP | 2734619 | A1 | 28 May 2014 |
| | | | | EP | 2734619 | B1 | 06 September 2017 |
| | | | | ES | 2653197 | T3 | 06 February 2018 |
| | | | | JP | 2014-524747 | A | 25 September 2014 |
| | | | | JP | 6275641 | B2 | 07 February 2018 |
| | | | | KR | 10-2014-0038530 | A | 28 March 2014 |
| | | | | MX | 2014000753 | A | 07 May 2014 |
| | | | | RU | 2014-101484 | A | 27 July 2015 |
| | | | | RU | 2565827 | C2 | 20 October 2015 |
| | | | | TW | 201311896 | A | 16 March 2013 |
| | | | | WO | 2013-010797 | A1 | 24 January 2013 |
| US | 2007-0196387 | A1 | 23 August 2007 | AT | 403674 | T | 15 August 2008 |
| | | | | AU | 2005-223371 | A1 | 29 September 2005 |
| | | | | AU | 2005-223371 | B2 | 09 June 2011 |
| | | | | AU | 2006-243854 | A1 | 09 November 2006 |
| | | | | CA | 2560185 | A1 | 29 September 2005 |
| | | | | CA | 2560185 | C | 07 May 2013 |
| | | | | DK | 1725581 | T3 | 10 November 2008 |
| | | | | EP | 1725581 | A1 | 29 November 2006 |
| | | | | EP | 1725581 | B1 | 06 August 2008 |
| | | | | EP | 1879912 | A1 | 23 January 2008 |
| | | | | ES | 2313310 | T3 | 01 March 2009 |
| | | | | NZ | 549776 | A | 31 July 2009 |
| | | | | US | 2006-0051742 | A1 | 09 March 2006 |
| | | | | US | 2010-0041022 | A1 | 18 February 2010 |
| | | | | US | 7638608 | B2 | 29 December 2009 |
| | | | | US | 7687611 | B2 | 30 March 2010 |
| | | | | US | 8283452 | B2 | 09 October 2012 |

INTERNATIONAL SEARCH REPORT
Information on patent family members

International application No.

**PCT/KR2021/004356**

| Patent document cited in search report | | | Publication date (day/month/year) | Patent family member(s) | | | Publication date (day/month/year) |
|---|---|---|---|---|---|---|---|
| | | | | WO | 2005-090390 | A1 | 29 September 2005 |
| | | | | WO | 2006-117354 | A1 | 09 November 2006 |
| KR | 10-2015-0060767 | A | 03 June 2015 | AU | 2013-325593 | A1 | 30 April 2015 |
| | | | | AU | 2013-325593 | B2 | 30 May 2019 |
| | | | | CA | 2886769 | A1 | 10 April 2014 |
| | | | | CN | 104797934 | A | 22 July 2015 |
| | | | | CN | 104797934 | B | 14 September 2018 |
| | | | | EP | 2905615 | A1 | 12 August 2015 |
| | | | | EP | 2905615 | A4 | 11 May 2016 |
| | | | | EP | 2905615 | B1 | 28 November 2018 |
| | | | | JP | 6280868 | B2 | 14 February 2018 |
| | | | | KR | 10-2094727 | B1 | 30 March 2020 |
| | | | | TW | 201430343 | A | 01 August 2014 |
| | | | | TW | I507688 | B | 11 November 2015 |
| | | | | US | 10365277 | B2 | 30 July 2019 |
| | | | | US | 2015-0233922 | A1 | 20 August 2015 |
| | | | | WO | 2014-054712 | A1 | 25 August 2016 |
| | | | | WO | 2014-054712 | A1 | 10 April 2014 |

Form PCT/ISA/210 (patent family annex) (July 2019)

**REFERENCES CITED IN THE DESCRIPTION**

**Patent documents cited in the description**

- KR 1020200052700
- KR 1020200052708
- KR 101464783
- WO 05108546 A
- WO 05104706 A
- WO 0510849 A
- KR 1020120024464
- KR 101370512